# EUROPEAN PATENT SPECIFICATION

(11) **EP 1 263 928 B1**
(45) Date of publication and mention of the grant of the patent: **26.09.2007**
(21) Application number: 01910649.1
(22) Date of filing: 12.02.2001
(51) Int. Cl.: C12N 5/22, G01N 33/574

(54) **IDENTIFICATION OF ANTIGENIC PEPTIDES BY CYTOTOXIC T LYMPHOCYTES ACTIVATED BY DENDRITIC CELL HYBRIDS**
IDENTIFIZIERUNG VON ANTIGENEN PEPTIDEN DURCH ZYTOTOXISCHE T-LYMPHOZYTEN AKTIVIERT VON DENDRITISCHEN ZELLHYBRIDEN
IDENTIFICATION DE PEPTIDES ANTIGENIQUES PAR DES LYMPHOCYTES T CYTOTOXIQUES ACTIVES PAR DES HYBRIDES DE CELLULES DENDRITIQUES

(30) Priority: 11.02.2000 US 181822 P; 24.02.2000 US 184687 P
(43) Date of publication of application: 11.12.2002
(73) Proprietor: DANA-FARBER CANCER INSTITUTE, INC., Boston, MA 02115 (US)
(72) Inventor: GONG, Jianlin, Wayland, MA 01778 (US); KUFE, Donald, W., Boston, MA 02115 (US)
(74) Representative: Crump, Julian Richard John
(86) International application number: PCT/US2001/004678
(87) International publication number: WO 2001/059073

(56) References cited:
- WO-A-00/57705
- WO-A-96/30030
- WO-A-98/46785
- TSAI V ET AL: "IDENTIFICATION OF SUBDOMINANT CTL EPITOPES OF THE GP 100 MELANOMA-ASSOCIATED TUMOR ANTIGEN BY PRIMARY IN VITRO IMMUNIZATION WITH PEPTIDE-PULSED DENDRITIC CELLS" JOURNAL OF IMMUNOLOGY,THE WILLIAMS AND WILKINS CO. BALTIMORE,US, vol. 158, no. 4, 1997, pages 1796-1802, XP000919288 ISSN: 0022-1767
- KAWASHIMA I ET AL: "IDENTIFICATION OF GP100-DERIVED, MELANOMA-SPECIFIC CYTOTOXIC PRIMARY IN VITRO IMMUNIZATION WITH PEPTIDE-PULSED DENDRITIC CELLS. T-LYMPHOCYTE EPITOPES RESTRICTED BY HLA-A3 SUPERTYPE MOLECULES BY" INTERNATIONAL JOURNAL OF CANCER,NEW YORK, NY,US, vol. 78, no. 4, 1998, pages 518-524, XP000917221 ISSN: 0020-7136
- GONG J., AVIGAN D., CHEN D., WU Z., KOIDO S., KASHIWABA M., KUFE D.: "Activation of antitumor cytotoxic T lymphocytes by fusions of human dendritic cells and breast carcinoma cells." PROC. NATL. ACAD. SCI. USA, vol. 97, no. 6, 14 March 2000 (2000-03-14), pages 2715-2718, XP002170124
- WANG J., SAFFOLD S., CAO X., KRAUSS J., CHEN W.: "Eliciting T cell immunity against poorly immunogenic tumors by immunization with dendritic cell-tumor fusion vaccines" J. IMMUNOL., vol. 161, 1998, pages 5516-5524, XP002171426
- GONG J ET AL: "INDUCTION OF ANTITUMOR ACTIVITY BY IMMUNIZATION WITH FUSIONS OF DENDRITIC AND CARCINOMA CELLS" NATURE MEDICINE,US,NATURE PUBLISHING, CO, vol. 3, no. 5, May 1997 (1997-05), pages 558-561, XP002910553 ISSN: 1078-8956
- GONG J., CHEN D. ET AL.: "Reversal of tolerance to human MUC1 antigen in MUC1 transgenic mice immunized with fusions of dendritic and carcinoma cells." PROC. NATL. ACAD. SCI. USA, vol. 95, May 1998 (1998-05), pages 6279-6283, XP002171428
- GONG J., NIKRUI N., CHEN D., KOIDO S. ET AL.: "Fusions of human ovarian carcinoma cells with autologous or allogeneic dendritic cells induce antitumor immunity." J. IMMUNOL., vol. 165, 1 August 2000 (2000-08-01), pages 1705-1711, XP002171429
- S A ROSENBERG ET AL: "Immunologic and therapeutic evaluation of a synthetic peptide vaccine for the treatment of patients with metastatic melanoma" NATURE MEDICINE,US,NATURE PUBLISHING, CO, vol. 4, no. 3, 1 March 1998 (1998-03-01), pages 321-327, XP002091661 ISSN: 1078-8956

## Description

The research described in this application was supported in part by grant no. CA38493 from the National Cancer Institute of the National Institutes of Health. The government may have certain rights in the invention.

### Field of the Invention

The invention relates to cellular immunology.

### Background of the Invention

Dendritic cells ("DC"s) are potent antigen-presenting cells ("APC"s) in the immune system. It has been shown that DCs provide all the signals required for T cell activation and proliferation. These signals can be categorized into two types. The first type, which gives specificity to the immune response, is mediated through interaction between the T-cell receptor/CD3 ("TCR/CD3") complex and an antigenic peptide presented by a major histocompatibility complex ("MHC") class I or II protein on the surface of APCs. This interaction is necessary, but not sufficient, for T cell activation to occur. In fact, without the second type of signals, the first type of signals can result in T cell anergy. The second type of signals, called costimulatory signals, is neither antigen-specific nor MHC-restricted, and can lead to a full proliferation response of T cells and induction of T cell effector functions in the presence of the first type of signals.

Costimulatory signals are generated by interaction between receptor-ligand pairs expressed on the surface of APCs and T cells. One exemplary receptor-ligand pair is one of the B7 costimulatory molecules on the surface of DCs and its counter-receptor CD28 or CTLA-4 on T cells (Freeman et al, Science 262: 909-911, 1993; Young et al., J. Clin. Invest. 90: 229, 1992; Nabavi et al., Nature 360: 266, 1992).

DCs are minor constituents of various immune organs such as spleen, thymus, lymph node, epidermis, and peripheral blood. For instance, DCs represent merely about 1% of crude spleen (Steinman et al., J. Exp. Med. 149:1, 1979) or epidermal cell suspensions (Schuler et al., J. Exp. Med. 161: 526, 1985; and Romani et al., J. Invest. Dermatol. 93: 600, 1989), and 0.1-1% of mononuclear cells in peripheral blood (Freudenthal et al., Proc. Natl. Acad. Sci. USA 87: 7698, 1990). Methods for generating dendritic cells from peripheral blood or bone marrow progenitors have been described (Inaba et al.. J. Exp. Med. 175: 1157, 1992; Inaba et al., J. Exp. Med. 176: 1693-1702, 1992; Romani et al., J. Exp. Med. 180: 83-93, 1994; and Sallusto et al., 1. Exp. Med. 179: 1109-1118, 1994).

### Summary of the Invention

The invention features methods of identifying peptides recognized by these CTL and HTL. Characterization of these peptides will allow identification of antigens (e.g., tumor associated antigens) that are the target of the immune response.

In methods of generating effector T lymphocytes (e.g., cytotoxic T lymphocytes (CTL) or helper T lymphocytes (HTL)) T lymphocytes are activated by fused cells (also referred to as "fusion cells") produced by fusion between mammalian (e.g., human) dendritic cells and non-dendritic cells (e.g., carcinoma cells) as described below. The T lymphocytes can be from, for example, a mammal (e.g., a human) with cancer (e.g., breast cancer) or an infectious disease (e.g., a viral disease such as AIDS). The fused cells have the characteristics described below. The CTL generated by this method lyse target cells (e.g., autologous tumor cells, pathogen infected cells) by a MHC restricted mechanism. Hit secrete helper cytokines that serve to enhance CTL and B-lymphocyte responses after activation by antigenic peptides bound to MHC molecules.

Fused cells are generated by fusion between at least one mammalian dendritic cell (e.g., a DC derived from a bone marrow culture or a peripheral blood cell culture) and at least one mammalian non-dendritic cell (e.g., a cancer cell or a transfected cell) that expresses a cell-surface antigen (e.g., a cancer antigen). At least 5% of a plurality of cells can be fused cells. By "cancer antigen" is meant an antigenic molecule that is expressed primarily or entirely by cancer cells, as opposed to normal cells, in an individual bearing the cancer. The fused cells in the compositions express, in an amount effective to stimulate an immune system (e.g., to activate T cells), MHC class II molecules, B7, and the cell-surface antigen. By "B7" is meant any member (e.g., B7-1 or B7-2) of the B7 family of costimulatory molecules.

The parental cells used to generate the fused cells can be obtained from a single individual (e.g., a human, a mouse, or a rat). They can also be obtained from different individuals of the same species (e.g., *homo sapiens*), with matching or non-matching MHC molecules. For example, the dendritic cells are autologous or allogeneic

Mammalian dendritic cells are fused with mammalian non-dendritic cells expressing a cell-surface antigen in the presence of a fusion agent (e.g., electricity, polyethylene glycol or Sendai virus). After optionally culturing the post-fusion cell mixture in a medium (which optionally contains hypoxanthine, aminopterin and thymidine) for a period of time, the cultured fused cells are separated from unfused parental non-dendritic cells, based on the different adherence properties of the two cell groups. The unfused parental dendritic cells do not proliferate, and so die off. Even if they remain present in the therapeutic composition, they will not interfere with the effects of the fused cells. The isolated fused cells, which typically express (a) MHC class II protein, (b) B7, and (c) the cell-surface antigen on the non-dendritic parental cells, are useful for stimulating an immune system. The cells are useful in screening methods or in therapeutic methods directly after fusion or after an *in vitro* culture step.

The DC phenotype of a fused cell can be maintained by re-fusing it with at least one additional mammalian dendritic cell. The re-fused cells express MHC class II molecules, B7, and the cell-surface antigen of the dendritic parental cells, and are useful for stimulating an immune system.

A method of making a population of cells containing CTL or HTL includes the steps of: (a) providing a plurality of cells which includes fused cells, each of which fused cells is generated by fusion between at least one mammalian dendritic cell and at least one mammalian non-dendritic cell (e.g., a cancer cell such as a human breast cancer cell) that expresses a cell-surface antigen, wherein at least 5% of the fused cells express, in amount effective to stimulate an immune response, (i) a MHC class II molecule, (ii) B7, and (iii) the cell-surface antigen; and (b) contacting a population of T lymphocytes with the plurality of cells, wherein the contacting causes differentiation of either (i) CTL precursor cells in the population of T lymphocytes to CTL; or (ii) HTL precursor cells in the population of T lymphocytes to HTL. In the plurality of cells provided, at least 5% of the cells are fused cells.

Also embraced by the invention is a method of testing a peptide for antigenic activity that includes the steps of: (a) providing the above population of cells containing CTL; and (b) contacting a plurality of target cells with the population of cells in the presence of the peptide. Lysis of the plurality of target cells or a portion of the plurality of target cells identifies the peptide as an antigenic peptide that is recognized by CTL within the population of cells. Alternatively, the method can include the steps of: (a) providing the above population of cells containing HTL; and (b) containing a plurality of antigen presenting cells with the population of cells in the presence of the peptide. A response (e.g., a proliferative response or a cytokine-producing response) of the HTL identifies the peptide as an antigenic peptide that is recognized by HTL within the population of cells. The mammalian non-dendritic cells used for producing the fused cells can be cancer cells (e.g., human breast cancer cells). In methods of inducing immune responses in subjects (e.g., patients such as human breast cancer patients), peptides identified by this method, or polypeptides of which the peptides are fragments, can be administered to the subjects.

A method for producing an antigen-specific immune effector cell is carried out by contacting a T cell with the hybrid cell described above. The T cell is derived from a variety of sources such as peripheral blood or from a tumor site. The contacting step occurs *in vivo* or *ex vivo.* For example, a method for producing a purified population of activated immune effector cells specific for a target antigen is carried out by contacting a T cell with a hybrid cell for a period of time sufficient to activate said T cell and removing the hybrid cell from said T cell to yield a population of antigen-specific immune effector cells.

Unless otherwise defined, all technical and scientific terms used herein have the same meaning as commonly understood by one of ordinary skill in the art to which this invention belongs. Exemplary methods and materials are described below, although methods and materials similar or equivalent to those described herein can be used in the practice or testing of the present invention. All publications and other references mentioned herein are incorporated by reference in their entirety. In case of conflict, the present specification, including definitions, will control. The materials, methods, and examples are illustrative only and not intended to be limiting.

Other features and advantages of the invention will be apparent from the following drawings, detailed description, and from the claims.

### Brief Description of the Drawings

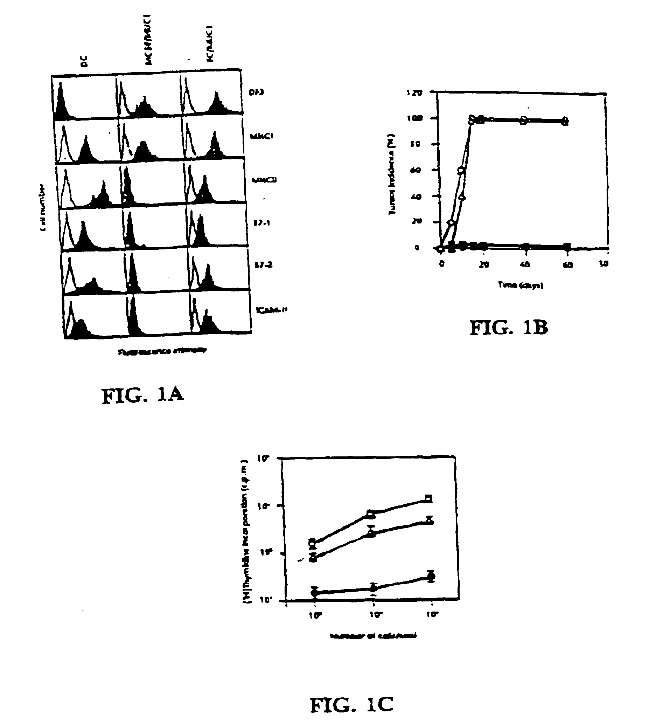

Fig. 1A is a graph showing the results of flow cytometric analysis of the indicated antigens on the surface of DCs (DC), MC38 cells (MC38/MUC1) and fused cells generated by fusion between DC's and MC38/MUC1 cells (FC/MUC1).
Fig. 1B is a graph showing tumor incidence in female C57BL/6 mice (10 per group) injected subcutaneously with 2 X 10⁵ MC38/MUC1 cells (open triangle), 2 X 10⁶ DCs mixed with 2 x 10⁵ MC38/MUC1 cells (open circle), 2 x 10⁵ FC/MUC1 cells (shaded circle), or 5 x 10⁵ FC/MUC1 cells (shaded box). Tumor incidence (> 3mm in diameter) was monitored at the indicated days after injection. Similar results were obtained in three separate experiments.
Fig. 1C is a graph showing [³H]-thymidine incorporation in mixed leukocyte reactions. DCs (open circle), MC38/MUCl cells (shaded circle), and FC/MUC1 cells (open triangle) were irradiated (30 Gy) and added at the indicated ratios to 1 x 10⁵ allogeneic Balb/c T cells. [³H]-Thymidine uptake at 6 h of incubation is expressed as the mean ± s.e.m. of three determinations. Similar results were obtained in three separate experiments.

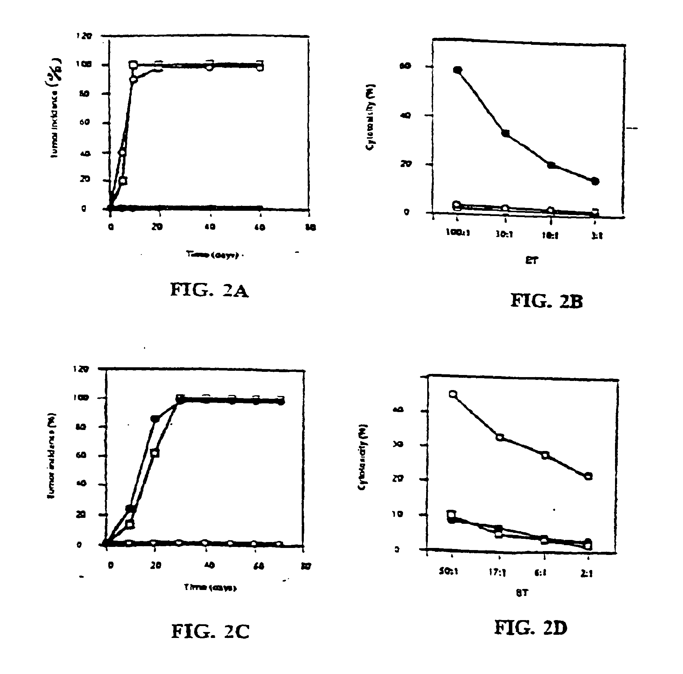

Fig. 2A is a graph showing induction of anti-tumor activity by FC/MUC1 in the form of percent tumor incidence. Groups of 10 mice were injected subcutaneously twice at 14-day intervals 3 x 10⁵ DC (open circle), 3 x 10⁵ FC/MUC1 (shaded circle), or PBS (open box). After 14 days, the mice were challenged subcutaneously with 2.5 x 10⁵ MC38/MUC1 cells. Tumors >3 mm in diameter were scored as positive. Similar results were obtained in three separate experiments.
Fig. 2B is a graph showing induction of anti-tumor activity by FC/MUC1 in the form of cytotoxicity. Mice injected twice with DC (open circle), FC/MUC1 (shaded circle) or PBS (open box) were challenged with 2.5 x 10⁵ MC38/MUC1 tumor cells. Splenocytes were isolated at 20 days after challenge and incubated at the indicated effector:target ratios with MC38/MUC1 target cells. Cytotoxic T lymphocyte ("CTL") activity (mean = s.e.m.) was determined by the 4-h LDH release assay. Similar results were obtained in three separate experiments.
Fig. 2C is a graph showing induction of anti-tumor activity by FC/MUC1 in the form of percent tumor incidence. Mice (8 per group) were injected intravenously and intraperitoneally every other day with mAbs against CD4⁺ (open box) and CD8⁺ (shaded circle) cells beginning 4 days before the first of two immunizations with FC/MUC1 and continuing until 4 days before challenge with 5 x 10⁵ MC38/MLTC1 cells. Rat IgG (open circle) was injected as a control. Tumors of >3 mm were scored as positive. Similar results were obtained in two separate experiments.
Fig. 2D is a line graph showing induction of anti-tumor activity by FC/MUC1 in the form of cytotoxicity. Mice were treated as above with mAbs against CD4⁺ (open box) and CD8⁺ (shaded circle), or rat IG (open circle), immunized with FC/MUC1 and then challenged with MC38/MUC1 cells. Splenocytes were harvested at 20 days after tumor challenge and incubated with MC38/MUC1 cells. CTL activity (mean ± s.e.m.) was determined by the 4-h LDH release assay. Similar results were obtained in three separate experiments.

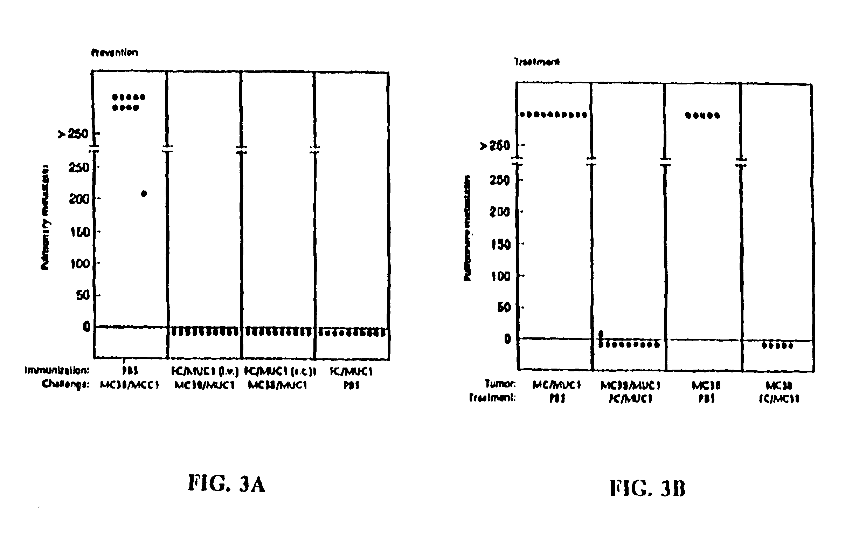

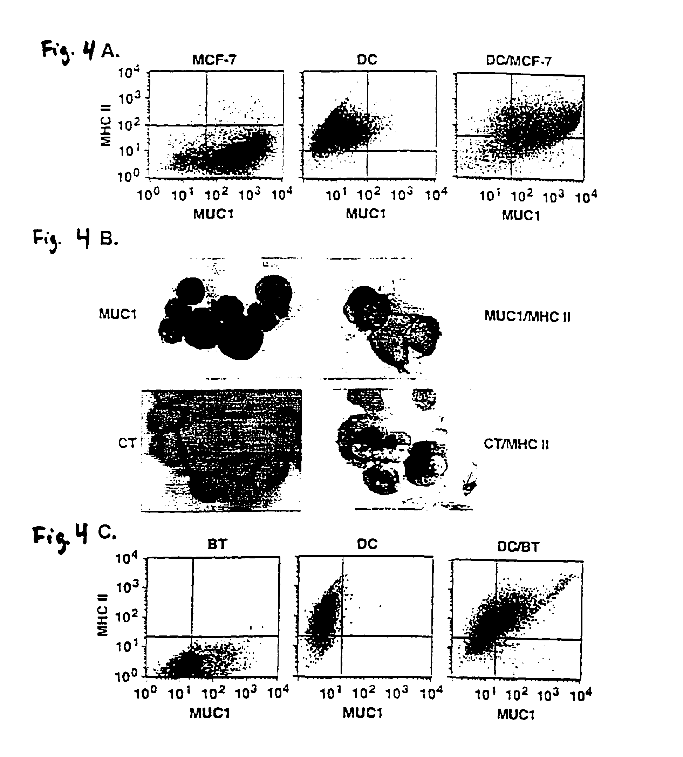

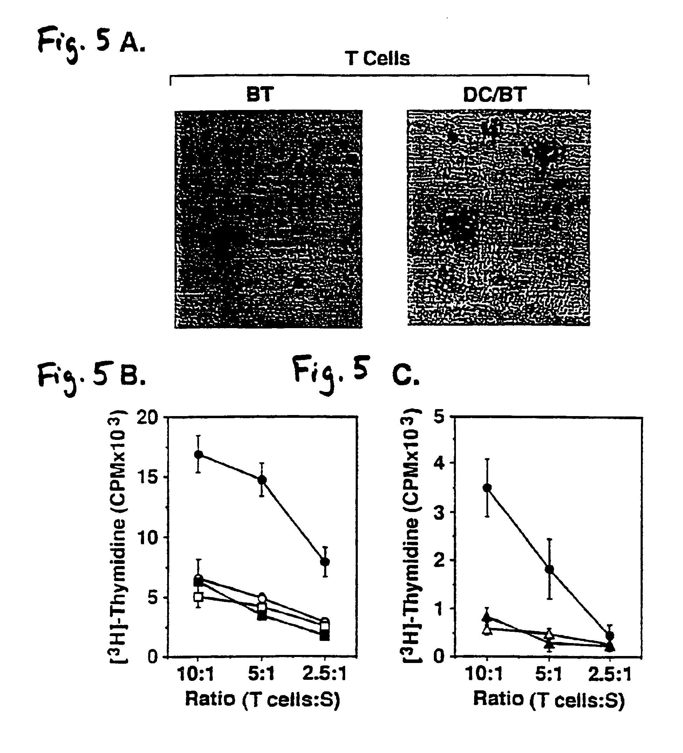

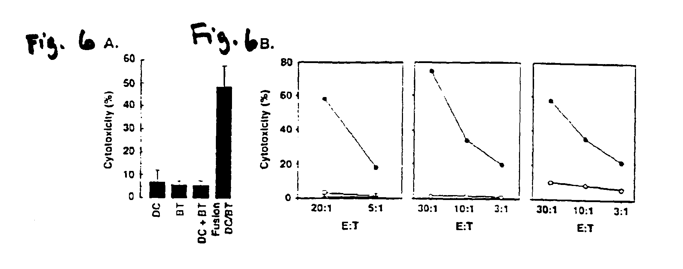

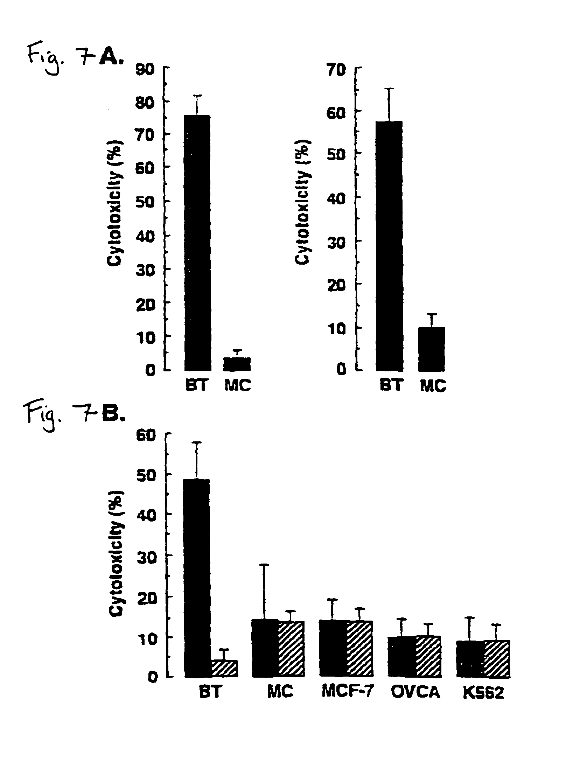

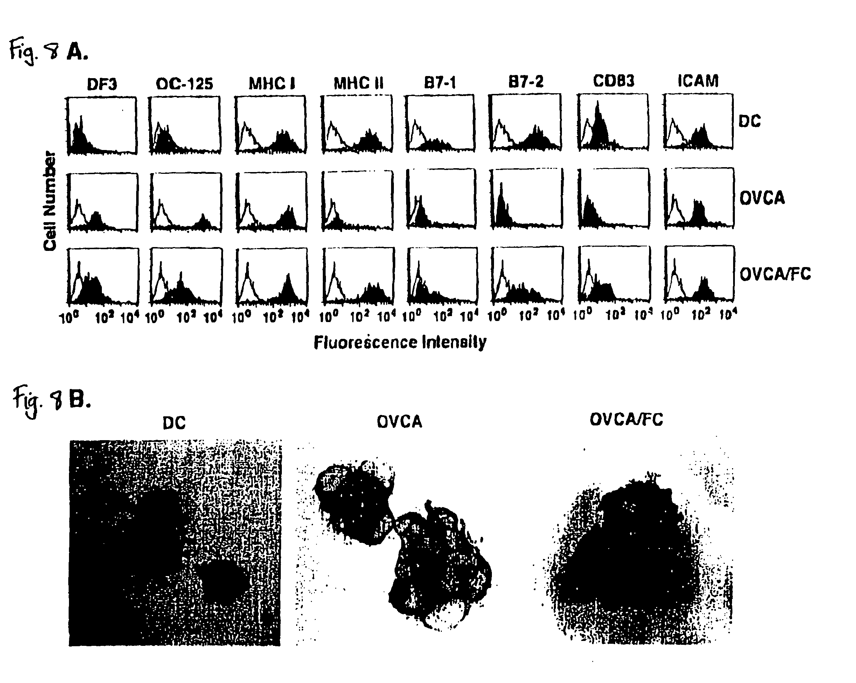

Fig. 3A is a graph showing prevention of MC38/MUC1 pulmonary metastases after immunization with FC/MUC1. Groups of 10 mice were injected twice with FC/MUC1 cells or PBS and then challenged after 14 days with intravenous administration of 1 x 10⁶ MC38/MUC1 cells. The mice were sacrificed 28 days after challenge. Pulmonary metastases were enumerated after staining the lungs with India ink (Wexler, J. Natl. Cancer Inst. 36: 641-643, 1966).
Fig. 3B is a graph showing treatment of MC38/MUC1 pulmonary metastases after immunization with FC/MUC1. Groups of 10 mice were injected intravenously with 1 x 10⁶ MC38/MUC1 cells or MC38 cells. The mice were immunized with 1 x 10⁶ FC/MUC1 or FC/MC38 at 4 and 18 days after tumor challenge and then sacrificed after an additional 10 days. Pulmonary metastases were enumerated for each mouse. Similar results were obtained in two separate experiments (10/10 mice treated with FC/MUC1 had no pulmonary metastases in the second experiment).

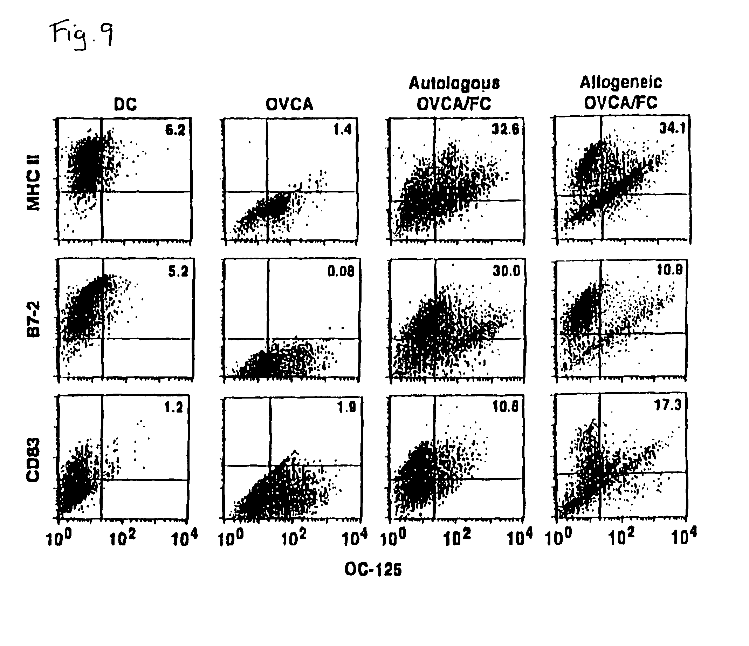

Fig. 4A is a series of bi-dimensional flow cytometry histograms showing expression of MUC1 and MHC class II on MCF-7 breast cancer cells, human dendritic cells, and fused DC/MCF-7 cells.
Fig. 4B is a series of photomicrographs showing expression of MUC1 (top left) and cytokeratin (CT) (bottom left) in primary human breast cancer cells and of MUC1 and MEC class II (top right) and cytokeratin and MHC class II (bottom right) in human DC/primary breast cancer fused cells.
Fig. 4C is a series of bi-dimensional flow cytometry histograms showing expression of MHC class II and MUC1 on primary human breast cancer cells (BT), autologous human dendritic cells (DC), and BT/DC fused cells.

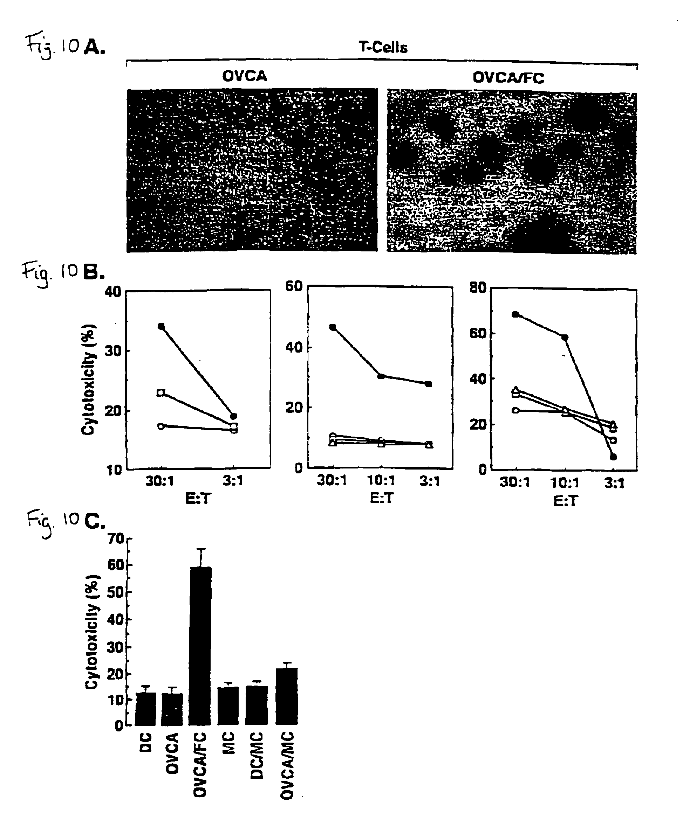

Fig. 5A is a pair of photomicrographs showing clustering of autologous T cells around BT/DC fused cells (right) but not BT cells (left).
Fig. 5B is a line graph showing the proliferation of T cells in response to stimulation with DC (open circle), autologous BT cells (open box), autologous BT cells mixed with autologous DC (shaded box), or autologous BT/DC fusion cells (shaded circle) at the indicated ratios of T cells to stimulator cells (S).
Fig. 5C is a line graph showing the proliferation of T cells in response to stimulation by PEG-treated autologous DC (open triangle), autologous DC fused to monocytes (shaded triangle), or autologous BT/DC fused cells (open circle).
Fig. 6A is a bar graph showing the cytolysis of autologous BT target cells by T cells stimulated, in the presence of human IL-2, with autologous DC, autologous BT cells, autologous DC mixed with autologous BT cells (DC + BT), or autologous DC/BT fused cells.
Fig. 6B is a set of three line graphs showing data obtained with cells from three different breast cancer patients. The graphs show the cytolysis of autologous BT target cells by T cells stimulated with either autologous BT cells (open circle) or DCBT fusion cells (shaded circle).

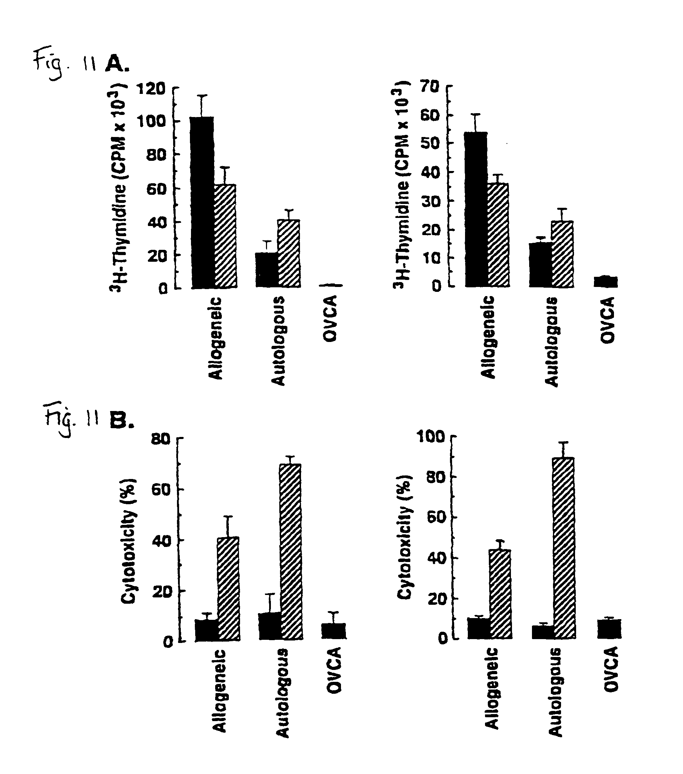

Fig. 7A is a pair of bar graphs showing data obtained with cells from two different breast cancer patients. The graphs show the cytolysis of autologous BT cells or autologous monocytes (MC) by T cells stimulated with autologous DCBT fused cells.
Fig. 7B is a bar graph showing the cytolysis, in the absence (solid bars) and presence (hatched bars) of antibody specific for human MHC class I molecules, of autologous BT cells, autologous MC, MCF-7 breast cancer cells, ovarian cancer cells (OVCA), and K562 cells by T cells stimulated with autologous DC/BT fused cells.
Fig. 8A is a series of flow cytometry histograms showing the expression of a variety of cell surface molecules on human DC, ovarian carcinoma cells (OVCA), and OVCA/DC fused cells (OVCA/FC).
Fig. 8B is a series of photomicrographs showing expression of HLA-DR (MHC class II) in DC. OVCA, and OVCA/DC fused cells (OVCA/FC).
Fig. 9 is a series of bi-dimensional flow cytometry histograms showing expression of CA-125 (OC-125), MHC class II (MHC II), B7-2, and CD38 on DC, OVCA cells, autologous OVCA/DC fused cells (autologous OVCA/FC), and allogeneic OVCA/DC fusion cells (allogeneic OVCA/FC).

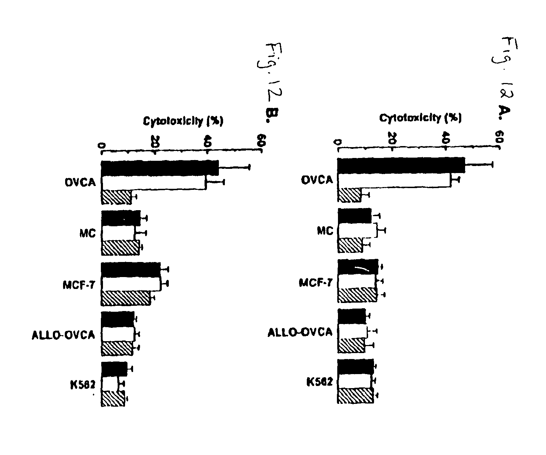

Fig. 10A is a pair of photomicrographs showing clustering of autologous T cells around OVCA/DC fused cells (OVCA/FC) (right) but not OVCA cells (left).
Fig. 10B is a set of three line graphs showing data obtained with cells from three different ovarian carcinoma patients. The graphs show the cytolysis of autologous OVCA target cells by T cells stimulated with either autologous DC (open circle), autologous OVCA cells (open box), autologous OVCA cells mixed with DC (open triangle), or OVCA/DC fused cells (shaded circle).
Fig. 10C is a bar graph showing cytolysis of autologous OVCA target cells by T cells stimulated with either autologous DC, autologous OVCA cells, autologous OVCA/DC fused cells (OVCA/FC), autologous monocytes (MC), autologous monocytes fused to autologous DC (DC/MC), or autologous OVCA cells fused to autologous monocytes (OVCA/MC).
Fig. 11A is a pair of bar graphs showing data obtained with cells from two different breast cancer patients. The graphs proliferative responses the T cells stimulated with autologous or allogeneic DC (solid bar) or OVAC/DC fused cells (hatched bar) produced by fusion of autologous OVCA cells with the autologous or allogeneic DC.
Fig. 11B is a pair of bar graphs showing data obtained with cells from two different breast cancer patients. The graphs show the cytolysis of autologous OVCA cells by T cells stimulated with autologous or allogeneic DC (solid bar), OVAC/DC fused cells (hatched bar) produced by fusion of autologous OVCA cells with the autologous or allogeneic DC, or autologous OVCA cells.
Fig. 12A is a bar graph showing the cytolysis, in the absence (solid bars) and presence (hatched bars) of antibody specific for human MHC class I molecules, of autologous OVCA cells, autologous monocytes (MC), MCF-7 breast cancer cells, allogeneic ovarian cancer cells (Allo-OVCA), and K562 cells by T cells stimulated with autologous OVCA/DC fused cells.
Fig. 12B is a bar graph showing the cytolysis, in the absence (solid bars) and presence (hatched bars) of antibody specific for human MHC class I molecules, of autologous OVCA cells, autologous monocytes (MC), MCF-7 breast cancer cells, allogeneic ovarian cancer cells (Allo-OVCA), and K562 cells by T cells stimulated with allogeneic OVCA/DC fused cells.

### Detailed Description of the Invention

The invention features: methods of identifying antigenic peptides recognized by the CTL and/or HTL.

DCs can be obtained from bone marrow cultures, peripheral blood, spleen, or other appropriate tissue of a mammal using protocols known in the art. Bone marrow contains DC progenitors, which, upon treatment with cytokines such as granulocyte-macrophage colony-stimulating factor ("GM-CSF") and interleukin 4 ("IL-4"), proliferate and differentiate into DCs. Tumor necrosis factor (TNF) may also be used to promote maturity of DCs. DCs so obtained are relatively immature (as compared to, for instance, spleen DCs). GM-CSF/IL-4 stimulated DC express MHC class I and class II molecules, B7-1, B7-2, ICAM, CD40 and variable levels of CD83. As discovered by Applicants, these immature DCs are more amenable to fusion than the more mature DCs found in spleen. Peripheral blood also contains relatively immature DCs or DC progenitors, which can propagate and differentiate in the presence of appropriate cytokines such as GM-CSF and which can also be used in fusion.

The non-dendritic cells used in the invention can be derived from any tissue or cancer (e.g., breast cancer, lung, pancreatic cancer, prostate cancer, bladder cancer, neurological cancers, genitourinary cancers, hematological cancers, melanoma and other skin cancers, and gastrointestinal cancers) by well known methods and can be immortalized. Non-dendritic cells expressing a cell-surface antigen of interest can be generated by transfecting the non-dendritic cells of a desired type with a nucleic acid molecule that encodes a polypeptide comprising the antigen. Exemplary cell-surface antigens are MUC1, α-fetoprotein, gamma-fetoprotein, carcinoembryonic antigen, fetal sulfoglycoprotein antigen, ferroprotein, placental alkaline phosphatase, and leukemia-associated membrane antigen. Methods for transfection and identifying antigens are well known in the art.

Fusion between the DCs and the non-dendritic cells can be carried out with well-known methods such as those using polyethylene glycol ("PEG") Sendai virus, or electrofusion. The ratio of DCs to non-dendritic cells in fusion can vary from 1:100 to 1000:1, with a ratio higher than 1:1 being preferred where the nondendritic cells proliferate heavily in culture. After fusion, unfused DCs usually die off in a few days in culture, and the fused cells can be separated from the unfused parental non-dendritic cells by the following two methods, both of which yield fused cells of approximately 50% or higher purity, i.e., the fused cell preparations contain less than 50%, and often less than 30%, unfused cells.

If the non-dendritic cells die or at least fail to proliferate in the presence of a given reagent and this sensitivity can be overcome by the fusion with DCs, the post-fusion cell mixtures containing the fused as well as the parental cells may be incubated in a medium containing this reagent for a period of time sufficient to eliminate most of the unfused cells. For instance, a number of tumor cell lines are sensitive to HAT due to lack of functional hypoxanthine-guanine phosphoribosyl transferasc ("HGPRT"). Fused cells formed by DCs and these tumor cell lines become resistant to HAT, as the DCs contribute functional HGPRT. Thus, a HAT selection can be performed after fusion to eliminate unfused parental cells. Contrary to standard HAT selection techniques, the HAT selection generally should not last for more than 12 days, since Applicants find that lengthy culturing leads to loss of MHC class II protein and/or B7 costimulatory molecules on the fused cells.

The second method of separating unfused cells from fused cells is based on the different adherence properties between the fused cells and the non-dendritic parental cells. It has been found that the fused cells are generally lightly adherent to tissue culture containers. Thus, if the non-dendritic parental cells are much more adherent, e.g., in the case of carcinoma cells, the post-fusion cell mixtures can be cultured in an appropriate medium (HAT is not needed but may be added if it slows the growth of unfused cells) for a short period of time. Subsequently, the fused cells can be gently dislodged and aspirated off, while the unfused cells grow firmly attached to the tissue culture containers. Conversely, if the non-dendritic parental cells grow in suspension, after the culture period, they can be gently aspirated off while leaving the fused cells loosely attached to the containers. Fused cells obtained by the above-described methods typically retain the phenotypic characteristics of DCs. For instance, these fused cells express T-cell stimulating molecules such as MHC class II protein, B7-1, B7-2, and adhesion molecules characteristic of APCs such as ICAM-1. The fused cells also continue to express cell-surface antigens of the parental non-dendritic cells, and are therefore useful for inducing immunity against the cell-surface antigens. Notably, when the non-dendritic fusion partner is a tumor cell, the tumorigenicity of the fused cell is often found to be attenuated in comparison to the parental tumor cell.

In the event that the fused cells lose certain DC characteristics such as expression of the APC-specific T-cell stimulating molecules, they (i.e., primary fused cells) can be re-fused with dendritic cells to restore the DC phenotype. The re-fused cells (i.e., secondary fused cells) are found to be highly potent APCs, and in some cases, have even less tumorigenicity than primary fused cells. The fused cells can be re-fused with the dendritic or non-dendritic parental cells as many times as desired.

Fused cells that express MHC class II molecules, B7. or other desired T-cell stimulating molecules can also be selected by panning or fluorescence-activated cell sorting with antibodies against these molecules.

The fused cells of the invention can be used to stimulate the immune system of a mammal for treatment or prophylaxis of a disease. For instance, to treat a tumor (primary or metastatic) in a human, a composition containing fused cells formed by his own DCs and tumor cells can be administered to him, e.g., at a site near the lymphoid tissue. The composition may be given multiple times (e.g., three to five times) at an appropriate interval (e.g., every two to three weeks) and dosage (e.g., approximately 10⁵-10⁸, e.g., about 0.5 X 10⁶ to 1 X 10⁶, fused cells per administration). For prophylaxis (i.e., vaccination) against cancer, non-syngeneic fused cells such as those formed by syngeneic DCs and allogeneic or xenogeneic cancer cells, or by allogeneic DCs and cancer cells, can be administered. To monitor the effect of vaccination, cytotoxic T lymphocytes obtained from the treated individual can be tested for their potency against cancer cells in cytotoxic assays. Multiple boosts may be needed to enhance the potency of the cytotoxic T lymphocytes. Example I below demonstrates that fusion cells formed by tumor cells and syngeneic DCs can prevent and treat tumors in animal models. Example III further demonstrates that such fusion cells may even activate anergized T cells that are specific for tumor antigens.

Cells infected with an intracellular pathogen can also be used as the nondendritic partner of the fusion for treatment of the disease caused by that pathogen. Examples of pathogens include, but are not limited to, viruses (e.g., human immunodeficiency virus, hepatitis A, B, or C virus, papilloma virus, herpes virus, or measles virus), bacteria (e.g., *Corynebacterium diphtheria. Bordetella petussis*), and intracellular eukaryotic parasites (e.g., *Plasmodium spp., Schistosoma spp., Leishmania spp., Trypanosoma spp., or Mycobacterium leprae*). Compositions containing the appropriate fused cells are administered to an individual (e.g., a human) in a regimen determined as appropriate by a person skilled in the art. For example, the composition may be given multiple times (e.g., three to five times) at an appropriate interval (e.g., every two to three weeks) and dosage (e.g., approximately 10⁵-10⁸, and preferably about 10⁷ fused cells per administration).

Alternatively, non-dendritic cells transfected with one or more nucleic acid constructs, each of which encodes one or more identified cancer antigens or antigens from a pathogen, can be used as the non-dendritic partner in fusion. These antigens need not be expressed on the surface of the cancer cells or pathogens, so long as the antigens can be presented by a MHC class I or II molecule on the fused cells. Fused cells generated by DCs and these transfected cells can be used for both treatment and prophylaxis of cancer or a disease caused by that pathogen. By way of example, fusion cells expressing MUC1 can be used to treat or prevent breast cancer, ovarian cancer, pancreatic cancer, prostate gland cancer, lung cancer, and myeloma; fusion cells expressing -fetoprotein can be used to treat or prevent hepatoma or chronic hepatitis, where - fetoprotein is often expressed at elevated levels; and fusion cells expressing prostate-specific antigen can be used to treat prostate cancer.

Methods of transfection and identifying antigens are well known in the art.

### Methods of Generating Effector Cells CTL and HTL

The effector cells (CTL and HTL) of the invention can be generated in a number of ways. Basically, lymphocytes (containing CTL and HTL precursor cells), e.g., in peripheral blood mononuclear cells (PBMC), spleen cell, or lymph node cells, are contacted with a composition containing any of the described fused cells of the invention. The contacting can be in vivo (e.g., by injecting the fused cells into the subject), *in vitro* (e.g., by culturing the cells containing lymphocytes with the fused cells), or by a combination of *in vivo* and *in vitro* methodologies (e.g., by *in vitro* activation of cells from a subject injected with the fused cells). The lymphocytes to be activated by the fused cells can be "virgin" T cells (i.e., T cells that have not, prior to being contacted with the fused cells, been exposed to activating antigens expressed by the fused cells) or they can have previously so exposed. For example, when the lymphocytes are in or from a patient with a tumor or an infectious disease, the lymphocytes will have been exposed to antigens expressed by the tumor or infected cells in the subject prior to the exposure to the fused cells.

The contacting with the fused cells can be single or multiple, e.g., a single *in vitro* exposure or multiple *in vitro* exposures, optionally after one or more in vivo exposures. The fused cells used for generation of effector cells can be used untreated or they can be pretreated with an agent (e.g., ionizing radiation or a drug such as mitomycin-C) that abrogates their proliferative capacity. In general, fused cells to be used for in vitro activation will have been pretreated with such an agent. The contacting can be performed with or without addition of an exogenous source of any of the cytokines exemplified below. The contacting of the precursor cells can result in activation of CTL and/or HTL. Testing of the activated cells for CTL and HTL activity can be performed using any of the methods described below.

Most CTL are CD8+ T cells restricted by MHC class I molecule and the majority of HTL are CD4+ T cells restricted by MHC class II molecules. However, CD4+ CTL restricted by MHC class II molecules and CD8+ HTL restricted by MHC class I molecules have also been described. In addition, there have also been reports of CD4+ CTL restricted by MHC class II molecules and CD4+ CTL restricted by MHC class I molecules. CD8+ CTL also produce, albeit at lower levels than CD4+ helper T cells, a wide range of cytokines. All these effector cell populations, provided they are generating by contacting of T lymphocytes with the fused cells of the invention, are encompassed by the invention. The CTL and HTL of the invention can be polyclonal or monoclonal. Methods of cloning CTL and HTL are known in the art.

### Method of Screening Candidate Peptide and Peptides for Antigenic Activity

The CTL and HTL ("effector cells") described above can be used to identify antigens expressed by the non-dendritic cell partners of the fused cells used to generate the effector cells of the invention, by a number of methods used in the art. In brief, the effector cell-containing cell population is cultured together with a candidate peptide or polypeptide and either an appropriate target cell (where cytotoxicity is assayed) or antigen presenting cell (APC) (where cell proliferation, or cytokine production is assayed) and the relevant activity is determined. A peptide that induces effector activity will be an antigenic peptide which is recognized by the effector cells. A polypeptide that induces effector activity will be an antigenic polypeptide, a peptide fragment of which is recognized by the effector cells.

Cytotoxic activity can be tested by a variety of methods known in the art (e.g., ⁵¹Cr or lactate dehydogenase (LDH) release assays described in Examples I and III-V). Target cells can be any of a variety of cell types, e.g., fibroblasts, lymphocytes, lectin (e.g., phytohemagglutinin (PHA), concanavalin A (ConA), or lipopolysaccbaride (LPS)) activated lymphocyte blasts, macrophages, monocytes, or tumor cell lines. The target cells should not naturally express the candidate antigens being tested for antigenic activity, though they could express them recombinantly. The target cells should, however, express at least one type of MEC class I molecule or MHC class II molecule (depending on the restriction of the relevant CU), in common with the CTL. The target cells can endogenously express an appropriate MHC molecule or they can express transfected polynucleotides encoding such molecules. The chosen target cell population can be pulsed with the candidate peptide or polypeptide prior to the assay or the candidate peptide or polypeptide can be added to the assay vessel, e.g., a microtiter plate well or a culture tube, together with the CTL and target cells. Alternatively, target cells transfected or transformed with an expression vector containing a sequence encoding the candidate peptide or polypeptide can be used. The CTL-containing cell population, the target cells, and the candidate peptide or polypeptide are cultured together for about 4 to about 24 hours. Lysis of the target cells is measured by, for example, release of ⁵¹Cr or LDH from the target cells. A peptide that elicits lysis of the target cells by the CTL is an antigenic peptide that is recognized by the CTL. A polypeptide that elicits lysis of the target cells by the CTL is an antigenic polypeptide, a peptide fragment of which is recognized by the CTL.

Candidate peptides or polypeptides can be tested for their ability to induce proliferative responses in both CU and HTL. The effector cells are cultured together with a candidate peptide or polypeptide in the presence of APC expressing an appropriate MHC class I or class II molecule. Such APC can be B-Iymphocytes, monocytes, macrophages, or dendritic cells, or whole PBMC. APC can also be immortalized cell lines derived from B-lymphocytes, monocytes, macrophages, or dendritic cells. The APC can endogenously express an appropriate MEC molecule or they can express a transfected expression vector encoding such a molecule. In all cases, the APC can, prior to the assay, be rendered non-proliferative by treatment with, e.g., ionizing radiation or mitomycin-C. The effector cell-containing population is cultured with and without a candidate peptide or polypeptide and the cells' proliferative responses are measured by, e.g., incorporation of [³H]-thymidine into their DNA.

As an alternative to measuring cell proliferation, cytokine production by the effector cells can be measured by procedures known to those in art. Cytokines include, without limitation, interleukin-2 (IL-2), IFN-, IL-4, IL-5, TNF-, interleukin-3 (IL-3), interleukin-6 (IL-6), interleukin-10 (IL-b), interleukin-12 (IL-12), interleukin-15 (IL-15) and transforming growth factor (TGF) and assays to measure them include, without limitation, ELISA, and bio-assays in which cells responsive to the relevant cytokine are tested for responsiveness (e.g., proliferation) in the presence of a test sample. Alternatively, cytokine production by effector cells can be directly visualized by intracellular immunofluorescence staining and flow cytometry.

Choice of candidate peptides and polypeptides to be tested for antigenicity will depend on the non-dendritic cells that were used to make the fused cells. Where the non-dendritic cells are tumor cells, candidate polypeptides will be those expressed by the relevant tumor cells. They will preferably be those expressed at a significantly higher level in the tumor cells than in the normal cell equivalent of the tumor cells. Candidate peptides will be fragments of such polypeptides. Thus, for example, for melanoma cells, the candidate polypeptide could be tyrosinase or a member of the MART family of molecules; for colon cancer, carcinoembryonic antigen; for prostate cancer, prostate specific antigen; for breast or ovarian cancer, HER2/neu; for ovarian cancer, CA-125; or for most carcinomas, mucin-1 (MUC1).

On the other hand, where the non-dendritic cells used to generate the fused cells were infected cells or cells genetically engineered to express a pathogen-derived polypeptide, the candidate polypeptide will be one expressed by the appropriate infectious microorganism or that expressed by the transfected cells, respectively. Examples of such polypeptides include retroviral (e.g., HIV or HTLV) membrane glycoproteins (e.g., gp160) or gag proteins, influenza virus neuraiminidase or hemagglutinin, *Mycobacterium tuberculosis or leprae* proteins, or protozoan (e.g., *Plasmodium* or *Trypanosoma*) proteins. Polypeptides can also be from other microorganisms listed herein. Peptides to be tested can be, for example, a series of peptides representing various segments of a full-length polypeptide of interest, e.g., peptides with overlapping sequences that, in tow, cover the whole sequence. Peptides to be tested can be any length. When testing MHC class I restricted responses of effector cells, they will preferably be 7-20 (e.g., 8-12) amino acids in length. On the other hand, in MHC class II restricted responses, the peptides will preferably be 10-30 (e.g., 12 - 25) amino acids in length.

Alternatively, a random library of peptides can be tested. By comparing the sequences of those eliciting positive responses in the appropriate effector cells to a protein sequence data base, polypeptides containing the peptide sequence can be identified. Relevant polypeptides or the identified peptides themselves would be candidate therapeutic or vaccine agents for corresponding diseases (see below).

Polypeptides and peptides can be made by a variety of means known in the art. Smaller peptides (less than 50 amino acids long) can be conveniently synthesized by standard chemical means. In addition, both polypeptides and peptides can be produced by standard *in vitro* recombinant DNA techniques, and *in vivo* genetic recombination (e.g., transgenesis), using nucleotide sequences encoding the appropriate polypeptides or peptides. Methods well known to those skilled in the art can be used to construct expression vectors containing relevant coding sequences and appropriate transcriptional/translational control signals. See, for example, the techniques described in Maniatis et al., Molecular Cloning: A Laboratory Manual [Cold Spring Harbor Laboratory, N.Y., 1989), and Ausubel et al., Current Protocols in Molecular Biology, [Green Publishing Associates and Wiley Interscience, N.Y., 1989).

A variety of host-expression vector systems can be used to express the peptides and polypeptides. Such host-expression systems represent vehicles by which the polypeptides of interest can be produced and subsequently purified, but also represent cells that can, when transformed or transfected with the appropriate nucleotide coding sequences, produce the relevant peptide or polypeptide *in situ.* These include, but are not limited to, microorganisms such as bacteria, e.g., *E*. *coli* or B. *subtilis,* transformed with recombinant bacteriophage DNA, plasmid or cosmid DNA expression vectors containing peptide or polypeptide coding sequences; yeast, e.g., *Saccharomyces* or *Pichia,* transformed with recombinant yeast expression vectors containing the appropriate coding sequences; insect cell systems infected with recombinant virus expression vectors, e.g., baculovirus; plant cell systems infected with recombinant virus expression vectors, e.g., cauliflower mosaic virus (CaMV) or tobacco mosaic virus (TMV), or transformed with recombinant plasmid expression vectors, e.g., Ti plasmids, containing the appropriate coding sequences; or mammalian cell systems, e.g., COS, CHO, BHK, 293 or 3T3, harboring recombinant expression constructs containing promoters derived from the genome of mammalian cells, e.g., metallothionein promoter, or from mammalian viruses, e.g., the adenovirus late promoter or the vaccinia virus 7.5K promoter.

Peptides of the invention include those described above, but modified for *in vivo* use by the addition, at either or both the amino- and carboxyl-terminal ends, of a blocking agent to facilitate survival of the relevant peptide *in vivo.* This can be useful in those situations in which the peptide termini tend to be degraded by proteases prior to cellular or mitochondrial uptake. Such blocking agents can include, without limitation, additional related or unrelated peptide sequences that can be attached to the amino and/or carboxyl terminal residues of the peptide to be administered. This can be done either chemically during the synthesis of the peptide or by recombinant DNA technology by methods familiar to artisans of average skill. Alternatively, blocking agents such as pyroglutamic acid or other molecules known in the art can be attached to the amino and/or carboxyl terminal residues, or the amino group at the amino terminus or carboxyl group at the carboxyl terminus can be replaced with a different moiety. Likewise, the peptides can be covalently or noncovalently coupled to pharmaceutically acceptable "carrier" proteins prior to administration.

Also of interest are peptidomimetic compounds that are designed based upon the amino acid sequences of the peptides or polypeptides. Peptidomimetic compounds are synthetic compounds having a three-dimensional conformation (i.e., a "peptide motif) that is substantially the same as the three-dimensional conformation of a selected peptide. The peptide motif provides the peptidomimetic compound with the ability to activate T cells in a manner qualitatively identical to that of the peptide or polypeptide from which the peptidomimetic was derived. Peptidomimetic compounds can have additional characteristics that enhance their therapeutic utility, such as increased cell permeability and prolonged biological half-life.

The peptidomimetics typically have a backbone that is partially or completely non-peptide, but with side groups that are identical to the side groups of the amino acid residues that occur in the peptide on which the peptidomimetic is based. Several types of chemical bonds, e.g., ester, thioester, thioamide, retroamide, reduced carbonyl, dimethylene and ketomethylene bonds, are known in the art to be generally useful substitutes for peptide bonds in the construction of protease-resistant peptidomimetics.

### Methods Using the Effector cells. Polypeptides, and Peptides of the Invention

The effector cells (CTL and HTL), polypeptides, and peptides of the invention can be used in basic research studies of tumor and infection immunology. They can be used in studies, for example, to further elucidate the mechanisms of antigen processing, antigen presentation, antigen recognition, signal transduction in CTL and HTL, and HTL-CTL interactions. In addition to other uses, they can be used as positive or negative controls in appropriate assays. They could also be used for diagnosis. For example, the ability of T cells from a test subject to respond to a polypeptide or peptide of the invention would be an indication that the test subject has or is susceptible to a disease associated with expression of the relevant peptide or polypeptide. CTL and HTL of the invention would be valuable "positive controls" for an appropriate diagnostic assay. Furthermore, the effector cells, polypeptides, and peptides can be used in methods of therapy and vaccination. These methods of the invention fall into 2 basic classes, i.e., those using *in vivo* approaches and those using *ex vivo* approaches.

### In Vivo Approaches

In one *in vivo* approach, a polypeptide, peptide or peptidomimetic can be administered to a subject by any of the routes listed above. It is preferably delivered directly to an appropriate lymphoid tissue (e.g. spleen, lymph node, or mucosal-associated lymphoid tissue (MALT)). The subject can have or be suspected of having any of the diseases disclosed herein. The immune response generated in the subject by administration of the polypeptide, peptide or peptidomimetic can either completely abrogate of decrease the symptoms of the disease. Alternatively, the polypeptide, peptide or peptidomimetic can be administered to a subject as a vaccine, i.e., with the object of preventing or delaying onset of a relevant disease.

The dosage required depends on the choice of polypeptide, peptide or peptidomimetic, the route of administration, the nature of the formulation, the nature of the patient's illness, and the judgment of the attending physician. Suitable dosages are in the range of 0.1-100.0 g/kg. Wide variations in the needed dosage are to be expected in view of the variety of polypeptides, peptides, or peptidomimetics of the invention available and the differing efficiencies of various routes of administration. For example, oral administration would be expected to require higher dosages than administration by i.v. injection. Variations in these dosage levels can be adjusted using standard empirical routines for optimization as is well understood in the art.

### Ex Vivo Approaches

In one *ex vivo* approach, populations of cells containing effector cells (CTL and/or HTL generated as described above using the fused cells of the invention) can be administered to a subject having or suspected of having any of the diseases described herein. The lymphoid cells used to generate the effector cells can have been obtained from the subject or a second subject, preferably of the same species, more preferably with no or a single MHC locus (class I or class II) incompatibility with the first subject. For example, donor lymphocyte infusion (DLI), in which allogeneic cells (e.g., PBMC) containing T lymphocytes arc infused into a subject, has been shown to decrease tumor load or even result in full remission in a variety of cancers. The therapeutic activity has been attributed to graft-versus-tumor activity of donor T-cells activated by MHC and/or non-MHC alloantigens of the recipient subject. The effector function of the cells used for DLI can be enhanced by exposing them (singly or multiply) (e.g., *in vitro*) to appropriate fused cells of the invention prior to infusion into the recipient subject. Preferably, but not necessarily, the fused cells will have been generated from dendritic cells and non-dendritic cells from the recipient subject. DLI is usually, but not necessarily, performed after nonmyeloablative bone marrow transplantation. DLI and non-myeloablative bone marrow transplantation methodologies are known in the art.

In a second *ex vivo* approach, lymphoid cells are isolated from the subject, or another subject; and are exposed (e.g., *in vitro*) to a polypeptide or peptide identified by the method of the invention in the presence of appropriate APC. The lymphoid cells can be exposed once or multiply (e.g., 2, 3, 4, 6, 8, or 10 times). The cytolytic, proliferative, or cytokine-producing ability of the stimulated lymphoid cells can be monitored after one or more exposures. Once the desired level of effector activity is attained, the cells can be introduced into the subject via any of the routes listed herein. Naturally, cells to be used for DLI (see above) could, instead of being activated by the fused cells of the invention, be activated by the peptides or polypeptides identified as described above.

Methods to test whether a peptide or polypeptide is therapeutic for or prophylactic against a particular disease are known in the art. Where a therapeutic effect is being tested, a test population displaying symptoms of the disease (e.g., cancer patients or experimental animals with cancer) is treated with a test effector cell-containing cell population, peptide, or polypeptide, using any of the above described strategies. A control population, also displaying symptoms of the disease, is treated, using the same methodology, with a placebo.

Disappearance or a decrease of the disease symptoms in the test subjects would indicate that the polypeptide or peptide was an effective therapeutic agent.

By applying the same strategies to subjects prior to onset of disease symptoms (e.g., human or experimental subjects with a genetic predisposition to the disease), effector cell-containing cell populations, polypeptides or peptides of the invention can be tested for efficacy in inducing immune responses or as prophylactic agents, i.e., vaccines. In this situation, prevention of or delay in onset of disease symptoms is tested. Alternatively, the levels of immune responses induced in the experimental arid control groups can be compared.

The following examples are meant to illustrate, but not limit, the invention.

### Example I. Fusion of Mouse Dendritic Cells and Non-Dendritic Cells

### Materials and Methods

### Cell culture and fusion

Murine (C57BL/6) MC38 adenocarcinoma cells were stably transfected with the DF3/MUC1 cDNA to generate the MC38/MUC1 cell line (Siddiqui et al., Proc. Natl. Acad. Sci. USA 85:2320-2323, 1988; Akagi et al., J. Immunother. 20:38-47, 1997). MC3S, MC38/MUC1 and the syngeneic MB49 bladder cancer cells were maintained in DMEM supplemented with 10% heat-inactivated fetal calf serum ("FCS"), 2 mM glutamine, 100 U/ml penicillin and 100 :g/ml streptomycin.

DCs were obtained from bone marrow culture using a method described by Inaba et al. (J. Exp. Med. 176: 1693-1702. 1992) with modifications. In brief, bone marrow was flushed from long bones, and red cells were lysed with ammonium chloride. Lymphocytes, granulocytes, and Ia⁺ cells were depleted from the bone marrow cells by incubation with the following monoclonal antibodies ("mAb"s):
(1) 2.43, anti-CD8 [TIB 210; American Type Culture Collection (ATCC), Rockville, MD];
(2) GK1.5, anti-CD4 (TIB 207, ATCC);
(3) RA3-3A1/6.1, anti B220/CD45R (TIB 146, ATCC);
(4) B21-2, anti-Ia (TIB 229, ATCC); and
(5) RB6-8C5, anti-Gr-I (Pharmingen, San Diego, CA);
and then complement. The unlysed cells were plated in six-well culture plates in RPMI 1640 medium supplemented with 5% heat-inactivated FCS, 50 :M 2-mercaptoethanol, 1 mM HEPES (pH 7.4), 2 mM glutamine, 10 U/ml penicillin, 10 :g/ml streptomycin and 500 U/ml recombinant murine GM-CSF (Boehringer Mannheim, Indiana). At day 7 of culture, nonadherent and loosely adherent cells were collected and replated in 100-mm petri dishes (10⁶ cells/ml; 8 ml/dish). The nonadherent cells were washed away after 30 mm of incubation and RPMI medium containing (JM-CSF was added to the adherent cells. After 18 hours in culture, the nonadherent cell population was removed for fusion with MC38IMTJCI cells or MC38.

Fusion was carried out by incubating cells with 50% PEG in Dulbecco's phosphate buffered saline ("PBS") without Ca²⁺ or Mg²⁺ at pH 7.4. The ratio of DCs to tumor cells in the fusion was from 15:1 to 20:1. After fusion, the cells were plated in 24-well culture plates in a medium containing HAT (Sigma) for 10-14 days. Because MC38 cells are not very sensitive to HAT, HAT was used to slow the proliferation of, rather than kill, MC38/MUC1 and MC38 cells. MC38/MUC1 and MC3S cells grow firmly attached to the tissue culture flask, while the fused cells were dislodged by gentle pipetting.

### Flow Cytometry

Cells were washed with PBS and incubated with mAb DF3 (anti-MUCI), mAb M1/42/3.9.8 (anti-MHC class I), mAb M5/114 (anti-MHC class II), mAb 16-10A1 (anti-B7-1), mAb GL1 (anti-B7-2) and MAb 3E² (anti-ICA.M-1) for 30 mm on ice. After washing with PBS, fluorescein isothiocyanate ("FITC")-conjugated anti-hamster. -rat and -mouse IgG was added for another 30 min on ice. Samples were then washed, fixed and analyzed by FACSCAN (Becton Dickinson, Mount View, CA).

### Cytotoxic T Cell Activity

Cytotoxic T cell ("CTL") activity was determined by the release of lactate dehydrogenase ("LDH") (CytoTox, Promega, Madison, WI).

### Mixed Leukocyte Reactions

The DCs, MC38/MUC1 and FC/MUC1 cells were exposed to ionizing radiation (30 Gy) and added to 1 x 10⁵ syngeneic or allogeneic T cells in 96-well flat-bottomed cultured plates for 5 days. The T cells were prepared by passing spleen suspensions through nylon wool to deplete residual APCs and plated to 90 mm in 100 mm tissue culture dishes. ³[H]-thymidine uptake in nonadherent cells was measured at 6 h after a pulse of 1 :Ci/well (GBq/mmol; Du Pont-New England Nuclear, Wilmington, DE). Each reaction was performed in triplicate.

### In Vivo Depletion of Immune Cell Subsets

Mice were injected both intravenously and intraperitoneally every other day with mAb GK1.5 (anti-CD4), mAb 2.43 (anti-CD8) or rat IgG 4 days before the first of two immunizations with FC/MUCI through 4 days before challenge with MC38/MUC 1 cells. The splenocytes were harvested for flow cytometry and analysis of CTL activity.

### Results

Murine MC38 adenocarcinoma cells were fused to bone marrow-derived DCs. To demonstrate successful fusions, MC38 cells that stably express the DF3/MUC1 tumor-associated antigen were first used (Siddiqui et al.. Proc. Natl. Acad. Sci. USA 75: 132-5136, 1978). The fusion cells (FC/MUCI) expressed DF3/MUCI, as well as MHC class I and II, B7-1, B7-2 and ICAM-1.

Moreover, most of the fusion cells exhibited a DC morphology with veiled processes and dendrites. Fusions of MC38 cells with DCs (FC/MC38) resulted in similar patterns of cell-surface antigen expression with the exception of no detectable DF3/MUC1 antigen. Injection of MC38/MUC1 cells in mice resulted in the formation of subcutaneous tumors. Similar findings were obtained with MC38/MUC1 cells mixed with DCs or after mixing MC38 cells with DCs.

However, the finding that no tumors formed in mice injected with FC/MUC1 indicated that the fusion cells are not tumorigenic.

Dendritic cells are potent stimulators of primary MLRs; Steinman et at, Proc. Natl. Acad. Sci. U.S.A. 75: 5132-5136, 1978; van Voorhis et al, J. Exp. Med. 158: 174-191, 1983) and induce the proliferation of allogeneic CD8 *T cells *in vitro* (Inaba et al., J. Exp. Med. 166: 182-194, 1987; Young et al., J. Exp. Med. 171: 1315-1332, 1990). To characterize in part the function of FC/MUCI cells, their effect in primary allogeneic MLRs was compared with the effect of DC and MC38/MUC1 cells. The results demonstrate that, like DCs, FC/MUC1 cells exhibit a stimulatory function in allogeneic MLR. By contrast, MC38/MUC1 cells had little effect on T cell proliferation.

Mice were immunized twice with FC/MUCI cells to assess *in vivo* function. Tumors developed in mice that had been immunized twice with 10⁶ irradiated MC38/MUC1 cells and subsequently challenged with MC38/MUC1 cells (Table I). In contrast, after immunization with 2.5 X 10⁵ FC/MUC1 cells, all animals remained tumor-free after challenge with MC38/MUCI cells (Table 1). Control animals immunized with DCs alone or PBS and then challenged subcutaneously with 2.5 X 10⁵ MC38 or MC38/MUC1 cells, however, exhibited tumor growth within 10-20 days.

Moreover, immunization with FC/MUCI or FC/MC3S had no detectable effect on growth of unrelated syngeneic MB49 bladder carcinoma (Table 1). CTLs from mice immunized with FC/MUCI cells induced lysis of MC38/MUCI, but not MB49 cells. By contrast, CTLs from mice immunized with DCs or PBS exhibited no detectable lysis of the MC38/MUCI targets.

To further define the effector cells responsible for antitumor activity, mice were injected intraperitoneally with antibodies against CD4⁺ or CD8⁺ cells before and after immunization with FC/MUC1. Depletion of the respective population by 80-90% was confirmed by flow cytometric analysis of splenocytes. The finding that injection of anti-CD4 and anti-CD8 antibodies increases tumor incidence indicated that both CD4⁺ and CD8⁺ T cells contributed to antitumor activity (Fig. 2C).

Moreover, depletion of CD4⁺ and CD8⁺ T cells was associated with reduced lysis of MC38/MUC1 cells in vitro (Fig. 2D).

To determine whether immunization with FC/MUC1 cells is effective for the prevention of disseminated disease, a model of MC38/MUC1 pulmonary metastases was used. Immunization with FC/MUC1 intravenously or subcutaneously completely protected against intravenous challenge with MC38/MUC1 cells. By contrast, all unimmunized mice similarly challenged with MC38/MUC 1 cells developed over 250 pulmonary metastases.

In a treatment model, MC38/MUC1 pulmonary metastases were established 4 days before immunization with FC/MUC1. While control mice treated with vehicle developed over 250 metastases, nine out of ten mice treated with FC/MUC1 cells had no detectable metastases and one mouse had fewer than 10 nodules. Mice treated with FC/MC3S cells similarly had no detectable MC38 pulmonary metastases. These findings indicated that FC/MUC1 immunization can be used for both the prevention and treatment of metastatic disease.

### Example II. Fusion of Human DCs and Myeloma Cells

### Materials and Methods

Leukocytes in buffy coats (or leukopacks) obtained by leukophoresis were fractionated by centrifugation in Ficoll. The fraction containing (peripheral blood) mononuclear cells was incubated in a flask containing RPMI 1640 supplemented with 10% fetal calf serum ("FCS") for 30 mm at 37°C. Nonadherent cells, some of which were dendritic cells, were gently separated from the adherent cells which were retained. To collect the DCs in the nonadherent population, the nonadherent cells were incubated in RPMI 1640 supplemented with 20% FCS for 30 mm to 1 hr, after which floating cells were removed and discarded. Both adherent cell samples were then incubated in RPMI 1640 supplemented with 20% FCS for 2-3 days to allow detachment of the loosely adherent cells (DCs). These loosely adherent cells were removed and retained. The remaining adherent cells, which still contained a relatively low proportion of loosely adherent DCs, were incubated with RPMI 1640 supplemented with 10% fetal calf serum overnight to allow detachment of the loosely adherent DCs. These were separated from the remaining adherent cells. The two samples of loosely adherent DCs were then pooled and cultured in medium containing GM-CSF (1000U/ml) and IL-4 (100U/ml) at a density of 10⁶ cells/ml for 5-6 days. The cells recovered from these cultures were the DCs used in fusion experiments.

DCs were also obtained from bone marrow stem cell cultures. In brief, stem cells were placed in a flask containing RPMI 1640 supplemented with 10% FCS. After 30 min of incubation at 37°C, nonadherent cells were washed away. Fresh RPMI 1640 supplemented with 10% FCS was added to the remaining, adherent cells. After overnight incubation, loosely adherent cells were collected and incubated in RPMI 1640/10% FCS medium containing GM-CSF (1000U/ml) and IL-4 (100U/ml) for 5-6 days. The resultant cells were DCs that were ready for use in fusion.

Cell fusion was carried out between DCs and human myeloma cells MY5 to produce fused cells DC/MY5. After fusion, the cells were placed in HAT selection for 10-14 days. IL-6 was also added to the culture at 20-50 :g/ml to promote survival of DC/MY5 cells. Procedures for fusion were essentially the same as described in Example 1, *supra,* except that the fused cells were separated from unfused myeloma cells based upon the higher degree of surface adherence exhibited by the fused cells.

### Results

As shown by flow cytometry, DC/MY5 cells retained the phenotypic characteristics of their parental cells: DC/MY5 were positively stained by mAbs for HLA-DR, CD38 (a myeloma cell-surface marker), DF3 (a tumor cell-surface marker), CD83 (a DC cell-surface marker), B7-1, and B7-2. Mixed lymphocyte reaction (MLR) assays demonstrated that these fused cells were also potent stimulators of T cells.

### Example III. Reversal of Tolerance to Human MUCI

### Antigen in MUC1 Transgenic Mice Immunized with Fusion Cells

### Materials and Methods

### MUC1 transgenic mice

A C57B1/6 mouse strain transgenic for human MUCI was established as described by Rowse et al. (Cancer Res. 58:315-321, 1998). 500 ng of tail DNA was amplified by PCR using MUCI primers corresponding to nucleotides 745 to 765 and nucleotides 1086 to 1065, respectively, to confirm the presence of MUCI sequences.

The PCR product was detected by electrophoresis in a 1% agarose gel (Rowse et al., supra).

### Cell culture and fusion

Murine (C57B1/6) MC38 and MB49 carcinoma cells were stably transfected with a MUC1 cDNA (Siddiqui et al., Proc. Natl. Acad. Sci. USA 85 :2320-2323, 1988; Akagi et al., J. Immunotherapy 20: 38-47, 1997; Chen et al., J. Immunol. 159:351-359, 1997). Cells were maintained in DMEM supplemented with 10% heat-inactivated FCS, 2 mM L-glutamine, 100 U/ml penicillin, and 100 g/ml streptomycin. DC were obtained from bone marrow culture and fused to the carcinoma cells as described in Example I, *supra.*

### In vitro T cell proliferation

Single cell preparations of spleen and lymph nodes were suspended in RPMI medium supplemented with 10% heat-inactivated FCS, 50 M -mercaptoethanol, 2 mM L-glutamine, 100 U/ml penicillin, and 100 g/ml streptomycin. The cells were stimulated with 5 U/ml purified MUC1 antigen (Sekine et al., J. Immunol, 135:3610-3616, 1985). After 1,3 and 5 days of culture, the cells were pulsed with 1 Ci [³H]thymidine per well for 12 hours and collected on filters with a semi-automatic cell harvester. Radioactivity was quantitated by liquid scintillation.

### Generation of CD8⁺ T cell Tines

Lymph node cells ("LNC") were suspended in complete RPMI medium containing 5 U/ml MUC 1 antigen. Ten U/ml murine IL-2 was added after 5 days of culture. On days 10 and 15, the cells were restimulated with 5 U/ml MUCI antigen and 1:5 irradiated (30 Gy) syngeneic spleen cells as APCs. T cell cultures were analyzed after removal of dead cells by Ficoll centrifugation and depletion of residual APCs by passage through nylon wool. The T cells were stained with FITC-conjugated antibodies against CD3e (145-2C11), CD4 (H129,19), CD8 (53-6.7), TcR (H57-597) and TcR (UC7- 13D5) (PharMingen). After incubation on ice for 1 hour, the cells were washed, fixed and analyzed by FACSCAN (Becton-Dickinson).

### Cytotoxicity assays

*In vitro* cytotoxicity was measured in a standard ⁵¹Cr-release assay. Briefly, cells were labeled with ⁵¹Cr for 60 minutes at 37°C and then washed to remove unincorporated isotope. The target cells (1x 10⁴) were added to wells of 96-well v-bottom plates and incubated with effector cells for 5 hours at 37°C. The supernatants were assayed for ⁵¹Cr in a gamma counter. Spontaneous release of ⁵¹Cr was assessed by incubation of target cells in the absence of effectors, while maximum or total release of ⁵¹Cr was determined by incubation of targets in 0.1% Triton-X-100. Percentage of specific ⁵¹Cr release was determined by the following equation: percent specific release = [(experimental-spontaneous)/(maximum-spontaneous))x100.

### Humoral immune responses

Microtiter plates were coated overnight at 4°C with 5 U/well purified MUCI antigen. The wells were washed with PBS containing 5% horse serum albumin and then incubated for 1 hour with four-fold dilutions of mouse sera. After washing and incubation with goat anti-mouse IgG conjugated to horseradish peroxidase (Amersham Life Sciences), antibody complexes were detected by development with o-phenylenediainine (Sigma) and measurement in an ELISA microplate autoreader EL310 at an OD of 490 nm.

### Immunohistology

Freshly removed tissues were frozen in liquid nitrogen. Tissue sections of 5 m in width were prepared in a cryostat and fixed in acetone for 10 minutes. The sections were then incubated with monoclonal antibody DF3 (anti MDC1), anti-CD4 (H129,19) or anti-CD8 (53-6.7) for 30 minutes at room temperature and then subjected to indirect immunoperoxidase staining using the VECTASTAIN ABC kit (Vector Laboratories).

### Results

As shown in Example 1, vaccines derived from fusions of DC and MC38/MUCI carcinoma cells (FC/MUCI) induce potent anti-tumor immunity. To assess the effects of vaccinating MUC1 transgenic mice with FC/MUC1, the mice were immunized twice with 5x10⁵ FC/MUC1 and, as controls, with 10⁶ irradiated MC38/MUC1 cells or PBS. After challenge with 10⁶ MC38 or MC38/MUC1 cells, all mice immunized with irradiated MC38/MUC1 cells or PBS developed tumors. By contrast, no tumor growth was observed in mice immunized with FC/MUCI. Immunization of the MUC1 transgenic mice with FC/MUC1 had no effect on growth of the unrelated MB49 bladder carcinoma (Chen et al., J. Immunol. 159:351-359, 1997). However, MB49 cells that express MUCI (MB49/MUJCI) failed to grow in, the FC/MUC1-immunized mice.

To extend these results, CTLs from the FC/MUC1-immunized mice were assayed for lysis of target cells. CTLs from MUC1 transgenic mice immunized with irradiated MC38/MUCI cells or PBS exhibited little if any reactivity against MC38/MUCI cells. By contrast, CTLs from the mice immunized with FC/MUCI induced lysis of MC38, MC38/MUC1 and MB49/MUC1, but not MB49, cells. As shown in wild-type mice (Example I, *supra*)*,* immunization with FC/MUCI induces immunity against MUCI and other unknown antigens on MC38 cells. Thus, the demonstration that MB49/MUC1, and not MB49, cells are lysed by CTLs confirms that FC/MUCI induces a MUC1-specific response. Further, immunization of the MUCI transgenic mice with FC/MUCI, but not irradiated MC38/MUC1 or PBS, induced a specific antibody response against MUC1.

To determine whether T cells from the MUCI transgenic mice can be primed to induce an anti-MUCI response, draining LNC were isolated from mice immunized with irradiated MC38/MUC1 cells or FC/MUC1. The LNC were stimulated with MUCI antigen in vitro. The results demonstrate that LNC from mice immunized with PBS or irradiated MC38/MUC1 cells fail to proliferate in the presence of MUCI antigen. In contrast. LNC from mice immunized with FC/MUC1 responded to MUC1 with proliferation. To confirm the induction of CTLs against MUC1, draining LNC were isolated from MUCI transgenic mice immununized with FC/MUCI and cultured in the presence of MUCI antigen and irradiated splenocytes. Cells were analyzed by FACSCAN at the beginning and at 10 to 15 days of culture. The results demonstrate the selection of a predominantly CD8⁺T cell population after incubation with MUCI antigen. Unlike naive T cells from unimmunized MUC1 transgenic mice, these CD8⁺ T cells exhibited specific CTL activity against MC38/MUC1 and MB49/MUCI targets. Collectively, the results suggest that immunization with FC/MUC1 reverses unresponsiveness to MUCI in the MUC1 transgenic mice.

The finding that unresponsiveness to MUCI can be reversed by immunization with FC/MUCI suggested that this vaccine could be used to treat disseminated disease in a background of MUC1 expression by normal epithelia. In a treatment model, MC38/MUC1 pulmonary metastases were established by tail vein injection of MC38/MUC1 cells into the MUC 1 transgenic mice. Whereas control mice treated with vehicle developed pulmonary metastases, mice immunized with FC/MUCI on day 2 or 4 had no detectable metastases. These findings indicates that PC/MUCI immunizations can be used to treat metastatic disease in the MUC1 transgenic mice. Importantly, mice protected against MC38/MUCI tumor exhibited persistent expression of MUCI antigen in normal bronchial epithelium and other tissues that express the transgene (Rowse et al., Cancer Res. 58:315-321, 1998). Also, staining of MUCI-positive tissues with anti-CD4 and anti-CD8 antibodies did not show any T cell infiltration.

Reversal of unresponsiveness against a self-antigen in adult mice has potential importance in the field of antitumor immunotherapy. The present example demonstrates that immunization with the DC-tumor fusion cells induces an immune response that is sufficient to achieve rejection of established metastases. Notably, induction of an anti-MUC1 response which confers anti-tumor immunity has little, if any, effect on normal secretory epithelia that express MUC 1 at apical borders along ducts. These findings demonstrate that the induction of anti-MUCI immunity represents an effective strategy for the treatment of MUCI-positive human tumors.

### Example IV. Activation of Tumor-specific CTL by Fusions of Human Dendritic Cells and Breast Carcinoma Cells

### Materials and Methods

### Breast carcinoma cell culture

Human MCF-7 breast carcinoma cells (ATCC, Rockville, MD) were grown in DMEM culture medium supplemented with 10% heat-inactivated FCS, 2 mL L-glutamine, 100 U/ml penicillin and 100:g/ml streptomycin. Human breast carcinoma cells were obtained with Institutional Review Board approval from biopsies of primary tumors and metastatic lesions of skin, lungs and bone marrow. The cells were separated by incubation in Ca2^{+/}Mg2⁺-free Hank's balanced salt solution containing 1 mg/ml collagenase, 0.1 mg/ml hyaluronidase and 1 mg/ml DNase. Breast tumor cells were also isolated from malignant pleural effusions by centrifugation and lysis of contaminating red blood cells. The breast tumor cells were maintained in RPMI 1640 medium supplemented with 10% heat -inactivated autologous-human serum, 2 mM L-glutamine, 100 U/ml penicillin. 100 :g/ml streptomycin and :g/ml insulin (Sigma).

### Preparation of DC, monocytes and T cells

Peripheral blood mononuclear cells (PBMC) were isolated from patients with metastatic breast cancer by Ficoll-Hypaque density gradient centrifugation. The PBMC were suspended in RPMI 1640 culture medium supplemented with 10% human serum (Sigma) for 1 h. The non-adherent cells were removed and T cells were isolated by nylon wool separation. The adherent cells were cultured for 1 week in RPMI 1640 medium/10% human serum containing 1000 U/ml GM-CSF (Genzyme) and 500 U/ml IL-4 (Genzyme). The GM-CSF/IL-4 stimulated DC expressed MHC class I and II, B7-1, B7-2, ICAM, CD40 and variable amounts of CD83, but not CD14, CD19, cytokeratin or MUC1. Non-adherent and loosely adherent cells were harvested by repeated washes to generate the DC population. Firmly adherent monocytes were released from the plates with trypsin.

### Cell fusion

DCs were mixed with MCF-7 or primary breast cancer cells at a 10:1 ratio and incubated in serum-free RPMI 1640 medium containing 50% polyethyleneglycol (PEG) for mm. After slowly diluting with serum-free RPMI 1640 medium, the cells were washed, resuspended in RPMI 1640 medium supplemented with 10% autologous human serum and 500 U/ml GM-CSF, and incubated at 37°C for 7-14 d.

### Flow cytometry

Cells were washed with PBS and incubated with murine antibodies directed against MUCI (DF3) (Kufe et al., Hybridoma 3:223-232, 1984), MHC class I (W6/32), MHC class II (HLA-DR). B7-1 (CD80), B7-2 (CD86) or ICAM (CD54) (Pharmingen) for 1 h on ice. After washing with PBS. the cells were incubated with fluorescein-conjugated goat anti-mouse IgG for 30 mm on ice. The cells were washed again and then incubated with PE-conjugated anti-MHC class II or anti-B7.1 for 1 h at 4°C. Samples were then washed, fixed with 2% paraformaldehyde and subjected to bi-dimentional analysis by FACScan (Becton-Dickinson, Mountain View, CA).

### Immunohistochemistry

Cytospin preparations of the cell populations were fixed in acetone for 10 min. The slides were incubated with MAb DF3 (anti-MUC1) or anti-cytokeratin antibody (AE1/AE3, Boehringer Mannheim, IN) for 30 mm at room temperature and then with biotinylated horse anti-mouse Ig for an additional 30 min. Reactivity was detected with ABC solution (Vector Laboratories, Burlingame, CA). The cells were then incubated with murine anti-MHC class II for 30 min and alkaline phosphatase labeled anti-mouse Ig for an additional 30min. AP-ABC solution (Vector) was used to generate a blue counterstain.

### Autologous T cell stimulation

DC, breast tumor cells and fusion cells were exposed to 30 Gy ionizing radiation and added to autologous T cells in 96-well, flat-bottom culture plates for 5d. [³H]-thymidine uptake by T cells was measured at 12 h after a pulse of I Ci/well (New England Nuclear, Wilmington, DE).

### CTL assays

PBMC were cocultured with autologous breast tumor or fusion cells for 10 d in the presence of 20 U/ml human interleukin-2 (HuIL-2). The stimulated T cells were harvested by nylon wool separation and used as effector cells in CTL assays with cell targets. Primary breast tumor cells, monocytes, MCF-7 cells, primary ovarian cancer cells (OVCA) and K562 cells were labeled with ⁵¹Cr for 60 mm at 37°C. After washing to remove unincorporated isotope, the targets (2 x 10⁴) were cocultured with effector cells for 5 h at 37°C. In the indicated experiment, labeled target cells were incubated with MAb Wb/32 (anti-MHC class I) for 30 min at 37°C before addition to the effector cells. The supernatants were assayed for ⁵¹Cr release in a gamma counter. Spontaneous release of ⁵¹Cr was assessed by incubation of targets in the absence of effectors, while maximum or total release of ⁵¹Cr was determined by incubation of targets in 0.1% Triton X-100. Percentage of specific ⁵¹Cr release was determined by the following equation: percent specific release = [(experimental - spontaneous) / (maximum - spontaneous)] x 100.

### Results

### Phenotype of human breast tumor/DC fusions

To determine whether human DCs can be used in the generation of heterokaryons with tumor cells, DC from PBMC of patients with metastatic breast cancer were prepared. The DCs were initially fused to human MCF-7 breast carcinoma cells. Bi-dimensional flow cytometry demonstrated that MCF-7 cells express the MUC1 carcinoma-associated antigen and MHC class I, but not MHC class II-B7-1, B7-2 or ICAM. By contrast, DC expressed MHC class I, class II and costimulatory molecules, but not MUC1. Following fusion of MCF-7 cells and DC, the resulting heterokaryons coexpressed MUC1 and MHC class II. Similar patterns of coexpression of MUC1 with B7. 1, B7-2 and ICAM were observed on the fused cells. Since these findings indicated that it was possible to generate of breast cancer cell/DC fused cells, human breast cancer cells were isolated from patients with primary or metastatic tumors for the purpose of making DC fusion cells with them. Immunostaining of short term cultures demonstrated that the breast carcinoma cells expressed MUCI and cytokeratin (CT). The breast tumor cells had no detectable expression of MHC class II, costimulatory or adhesion molecules. The tumor cells were fused with autologous DC and, after culturing for 7 days, the resulting population was analyzed for the presence of fusion cells. Fusion of the tumor cells to autologous DC resulted in the generation of heterokaryons that expressed both MUCI and MHC class II or cytokeratin and MHC class II. Analysis by bi-dimensional flow cytometry confirmed that the breast tumor cells (BT) express MUCI, and not MHC class II, while the autologous DC expressed MHC class II, but not MUC1. By contrast, over 40% of the fused cells (DC/BT) expressed both MUC1 and MHC class II. Similar results obtained by histochemical staining and bi-dimensional flow cytometry further indicated the presence of fusion cells and not aggregates. As assessed by both methods, the efficiency of autologous fusions prepared from six separate breast cancer patients ranged from 30 to 50% of the tumor cell population.

### Function of the breast tumor/DC fusions

To determine whether the autologous fusion cells are effective in stimulating autologous T cells, the heterokaryons were cocultured with T cells isolated from nonadherent PBMC. As a control, the T cells were also cocultured with autologous tumor cells. While there was no evidence for a T cell response to autologous tumor, the fusion cells stimulated T cell proliferation and the formation of T cell/fusion cell clusters. To assess the specificity of this response, autologous T cells were incubated with DC, irradiated breast tumor cells, a mixture of unfused DC and breast tumor cells, or DC-breast tumor fusion cells. There was little if any T cell stimulation by autologous DC, tumor or a mixture of the two cell types. As additional controls, autologous T cells exhibited little if any response to PEG-treated DC or DC fused to monocytes, as compared to the response obtained with DC/tumor fusion cells. These findings demonstrate that fusion of breast tumor cells and DC results in stimulation of a specific T cell response.

### Generation of CTL against human breast tumor

To assess the induction of tumor-specific CTL, T cells were stimulated for 10 d and then isolated for assaying lysis of autologous tumor cells. T cells incubated with autologous DC, irradiated breast tumor cells or an unfused mixture of both exhibited a low level of autologous breast tumor cell lysis. Significantly, T cells stimulated with the fusion cells were effective in inducing cytotoxicity of autologous tumor. Similar results were obtained with T cells from three breast cancer patients that had been stimulated with autologous DC/breast tumor cell fusions. Moreover, unstimulated T cells that had been cocultured with autologous breast tumor cells failed to mediate significant tumor cell killing. To define the specificity of the CTL generated by incubation with fusion cells, we compared their ability to lyse autologous tumor and other cell types. The two graphs in Fig. 7A show data obtained with cells from two individual patients. Incubation of fusion-stimulated T cells with autologous breast tumor or monocytes demonstrated selectivity for lysis of the tumor cells. In addition, T cells stimulated with autologous fusion cells demonstrated significant lysis of autologous breast tumor cells, while lysis of MCF-7 cells, primary ovarian cancer cells and NK-sensitive K562 cells was similar to that obtained with autologous monocytes. The finding that preincubation of the targets with an antibody specific for MHC class 1 resulted in abrogation of autologous breast tumor cell lysis indicated that the killing was MHC class 1 restricted. By contrast, the antibody specific for MHC class I had little if any effect on lysis of the other cell types. Activation of tumor-specific CTL by fusions of human Dendritic Cells and Ovarian Carcinoma Cells

### Materials and Methods

### Isolation of peripheral blood mononuclear cells (PBMC)

Mononuclear cells were isolated from the peripheral blood of patients with ovarian cancer and normal donors by Ficoll-Hypaque density gradient centrifugation. The PBMC were cultured in RPMI 1640 culture medium containing 1% autologous serum for 1 h. The non-adherent cells were removed and the T cells purified by nylon wool separation. The adherent cells were cultured for 1 week in RPMI 1640 culture medium containing 1% autologous serum, 1000 U/ml GM-CSF (Genzyme) and 500 U/ml IL-4 (Genzyme). DC were harvested from the non-adherent and loosely adherent cells. The firmly adherent monocytes were harvested after treatment with trypsin.

### Preparation and fusion of ovarian carcinoma cells

Ovarian carcinoma (OVCA) cells obtained from primary tumors and malignant ascites were separated from other cells and non-cellular components in Hank's balanced salt solution (Ca⁺⁺/Mg⁺⁺ free) containing 1 mg/ml collagenase, 0.1 mg/ml hyaluronidase and 1 mg/ml DNase. The cells were cultured in RPMI 1640 culture medium supplemented with 10% heat-inactivated autologous human serum, 2 mM L-glutamine, 100 U/ml penicillin and 100 g/ml streptomycin until fusion. Autologous or allogeneic DC were incubated with the OVCA cells for 5 min at a ratio of 10:1 in serum-free RPMI 1640 medium containing 50% polyethylene glycol (PEG). RPMI 1640 culture medium was then added slowly to dilute the PEG. After washing, the cells were resuspended in RPMI 1640 culture medium supplemented with 10% autologous serum and 500 U/ml GM-CSF for 7-14d.

### Phenotype analysis

Cells were incubated with mouse monoclonal antibodies (Mab) directed against human DF3/MUCI (MAb DF3) (Kufe et al, Hybridoma 3:223-232, 1984), human CA-125 (MAb OC-125) (Bast et al., N Engl. J Med 309(15):883-887, 1983), human MHC class I (W6/32), human MHC class II (RLA-DR), human B7-1 (CD80), human B7-2 (CD86), human ICAM (CD54; Pharmingen) and human CD83 (Pharmingen) for 1 h on ice. After washing with PBS, the cells were incubated with fluorescein-conjugated goat antibody specific for mouse IgG for 30 min. For dual expression analysis, cells were incubated with MAb OC-125, washed and then incubated with phycoerythrin-conjugated antibody specific for MHC class II, B7-2 or CD83 for 1 h at 4°C. Samples were washed, fixed in 2% paraformaldehyde and analyzed by FACScan (Becton-Dickinson, Mountain View, CA).

### Immunohistochemical staining

Cytospin cell preparations were fixed in acetone and incubated with MAb OC-125 for 30 min at room temperature. The slides were washed and incubated with biotinylated horse antibody specific for mouse IgG for an additional 30 min. Staining (red color) was achieved with ABC solution (Vector Laboratories, Burlingame. CA). The slides were then incubated with murine antibody specific for human MHC class II for 30 min followed by alkaline phosphatase-labeled anti-mouse IgO. AP-ABC solution (Vector Laboratories) was used to generate a blue counterstain.

### T cell proliferation assays

Cells were exposed to 30 Gy ionizing radiation and added to T cells in 96-well flat-bottom plates for 5 d. Incorporation of [³H)-thymidine by the T cells was measured after incubation in the presence of 1 Ci/well for 12 h.

### Cytotoxicity assays

T cells were stimulated with the indicated cell preparations for 1 week in the presence of 20 U/ml HuIL-2. The T cells were harvested by nylon wool separation and used as effector cells in CTL assays. Autologous OVCA cells, allogeneic OVCA cells, autologous monocytes, MCF-7 breast carcinoma cells and K562 cells were labeled with ⁵¹Cr for 60 mm at 37°C. After washing, targets (2x10⁴) were cultured with the T cells for 5 h at 37°C. In certain experiments, the labeled target cells were incubated with MAb W6/32 (anti-MHC class I) for 30 min at 37°C before addition of the effector cells. Supernatants were assayed for ⁵¹Cr release in a gamma counter. Spontaneous release of ⁵¹Cr was assessed by incubation of the targets in the absence of effectors. Maximum or total release of ⁵¹Cr was determined by incubation of the targets in 0.1 % Triton X-100. Percentage of specific ⁵¹Cr release was determined by the following equation: percent specific release = [(experimental - spontaneous) / (maximum-spontaneous)] x 100.

### Results

### Characterization of ovarian carcinoma (OVCA) cells fused with autologous and allogeneic DC

DC were generated from patients with metastatic ovarian cancer and from normal volunteers. Adherent cells were isolated from PBMC and cultured in the presence of GM-CSF and IL-4 for 1 week. The resulting population was subjected to FACS analysis. The DC displayed a characteristic phenotype with expression of MHC class I class II, costimulatory molecules, CD83 and ICAM, but not the DF3/MUC1 or CA-125 carcinoma-associated antigens. By contrast, OVCA 5 cells isolated from a patient with metastatic ovarian cancer expressed MUCI, CA-125, MHC class I and ICAM, but not MHC class II, B7-1, B7-2 or CD83. Similar findings were obtained with OVCA cells obtained from primary ovarian tumors and from malignant ascites. Fusion of the OVCA cells to autologous DC (OVCA/FC) resulted in the generation of heterokaryons (OVCA/FC) that express the CA-125 and MUCI antigens, MHC class II, B7-1, B7-2 and CD83.

Moreover, the pattern of antigen expression was similar when the OVCA cells were fused to allogeneic DC. Immunostaining confirmed that the DC expressed MHC class II and not CA-125. Conversely, the OVCA cells expressed CA-125 arid not MHC class II. Analysis of the fusion cells (OVCA/FC) demonstrated expression of both antigens.

Bi-dimensional flow cytometry was used to assess the efficiency of the fusions. In contrast to DC, the OVCA cells expressed CA-125, but not MHC class II, B7-2 or CD83. Analysis of OVCA cells fused with autologous DC demonstrated that 32.6% of the population expressed both CA-125 and MHC class II. Assessment of CA-125 and B7-2 expression demonstrated that 30% of the autologous OVCA/FC expressed both antigens. Moreover, 10.8% of the autologous OVCA/FC population expressed both CA-125 and CD83. Fusion of the OVCA cells and allogeneic DC also resulted in cells coexpressing CA-125 and MBC class II, B7-2 or CD83. These findings demonstrate the formation of heterokaryons by fusing OVCA cells to autologous or allogeneic DC.

### Stimulation of anti-tumor CTL by autologous OVCA/FC

To assess the function of OVCA/FC, the fusion cells were cocultured with autologous PBMC. The experiment was performed with cells from three individual patients and each of the three graphs in Fig. 10B shows data obtained with cells from one of the patients. As a control, the PBMC were also cultured with autologous OVCA cells. The fusion cells, but not the tumor cells, stimulated the formation of T cell clusters. After 10 d of stimulation, the T cells were isolated for assessment of cytolytic activity. Using autologous OVCA cells as targets, there was a low level of lysis when assaying T cells that had been incubated with autologous DC, autologous tumor, or a mixture of unfused DC and tumor. By contrast, T cells stimulated with the OVCA/FC were effective in inducing lysis of autologous tumor targets. Similar results were obtained with T cells from the three patients with ovarian cancer. As a control, T cells stimulated with OVCA cells fused to autologous monocytes (OVCA/MC) or DC fused to monocytes (DC/MC) had little effect on stimulation of anti-tumor CTL activity.

### Generation of anti-tumor CTL by OVCA cells fused to allogeneic DC

To assess OVCA/FC function when the fusion is performed with allogeneic DC, autologous PBMC were stimulated with OVCA cells fused to autologous or allogeneic DC. Each of the two graphs in Figs. 11A and 11B shows data obtained with cells from an individual patient. As controls, the autologous PBMC were also stimulated with unfused DC or OVCA cells. Incubation of the T cells with allogeneic DC was associated with greater stimulation than that obtained with autologous DC. The results also demonstrate that T cell proliferation is stimulated to a greater extent by OVCA fused to allogeneic, as compared to autologous, DC. Similar findings were obtained with T cells obtained from the two patients. After stimulation for 10 d, the T cells were isolated and assessed for lysis of autologous tumor. Stimulation with unfused allogeneic or autologous DC had little if any effect on lytic function compared to that obtained with T cells stimulated in the presence of OVCA cells. By contrast, T cells stimulated with OVCA cells fused to allogeneic DC induced lysis of autologous tumor. Moreover, for both patients, T cells stimulated with OVCA cells fused to autologous or allogeneic DC exhibited induction of CTL activity. These findings demonstrate that the anti-tumor activity of autologous CTLs is stimulated by fusions of tumor cells to autologous or allogeneic DC.

### Specificity of OVCA/FC-stimulated CTLs

To assess the specificity of CTL induced by fusion cells, T cells stimulated with OVCA cells fused to autologous DC were incubated with autologous tumor, autologous monocytes, MCF-7 breast carcinoma cells, allogeneic OVCA cells and NK-sensitive K562 cells. The data shown in Figs. 12A and 12B were obtained from CTL assay cultures carried out in the absence (solid bars) or presence (hatched bars) of MAb specific for human MHC class I molecules. Incubation of the OVCA/FC stimulated T cells, with autologous tumor or monocytes demonstrated selective lysis of the tumor. In addition, there was no significant lysis of the MCF-7, allogeneic OVCA or K562 cells by these CTL. Preincubation of the targets with an anti-MHC class I antibody blocked lysis of the autologous OVCA cells and had little effect on that obtained for the other cell types in the absence of antibody. T cells stimulated with autologous OVCA cells fused to allogeneic DC also demonstrated selective lysis of the autologous tumor. Moreover, lysis of the autologous tumor was abrogated by preincubation of the targets with anti-MHC class I, thereby indicating that recognition of the tumor by the CTL was restricted by MHC class I molecules.

### Other Embodiments

It is to be understood that while the invention has been described in conjunction with the detailed description thereof, the foregoing description is intended to illustrate and not limit the scope of the invention, which is defined by the scope of the appended claims.

## Claims

1. A method of testing a peptide for antigenic activity, the method comprising:
(a) providing a hybrid cell comprising a fusion product of a human dendritic cell and a human tumor or cancer cell, wherein said tumor or cancer cell is a primary or metastatic tumor cell, wherein said hybrid cell expresses B7 on its surface;
(b) contacting the hybrid cell with a human immune effector cell, thereby producing a human educated immune effector cell; and
(c) contacting a target cell with said human educated immune effector cell in the presence of the peptide, wherein lysis of said target cell identifies the peptide as an antigenic peptide.

2. A method as claimed in claim 1, comprising:
(a) providing a plurality of cells, wherein at least 5% of the cells of said population are fused cells generated by fusion between at least one human dendritic cell and at least one human tumor or cancer cell that expresses a cell-surface antigen, wherein said tumor or cancer cell is a primary or metastatic tumor cell, wherein said fused cells express, in amounts effective to stimulate an immune response, (i) a MHC class II molecule, (ii) B7, and (iii) the cell-surface antigen,
(b) contacting a population of human T lymphocytes with the plurality of cells, wherein the contacting causes differentiation of effector cell precursor cells in the population of T lymphocytes to effector cells comprising cytotoxic T lymphocytes; and
(c) contacting a plurality of target cells with the effector cells in the presence of the peptide;
wherein lysis of the plurality of target cells or a portion thereof identifies the peptide as an antigenic peptide that is recognized by the cytotoxic T lymphocytes.

3. The method of claim 1, wherein the peptide is a fragment of a protein expressed by the cancer cell.

4. A method comprising the steps of testing one or more peptides for antigenic activity in accordance with claim 1, identifying at least one of said peptides as an antigenic peptide, and using said antigenic peptide or a polypeptide of which said antigenic peptide is a fragment in the manufacture of a medicament for use in inducing an immune response in a subject.

5. A method as claimed in claim 4, wherein said peptide is derived from a polypeptide selected from the group consisting of carcinoembryonic antigen, prostate specific antigen, HER2/neu, CA-125, and mucin-1 (MUC1).

## Patentansprüche

1. Verfahren zum Testen eines Peptids auf antigenische Aktivität, wobei das Verfahren folgendes umfasst:
a) Bereitstellen einer hybriden Zelle, die ein Fusionsprodukt einer menschlichen dentritischen Zelle und einer menschlichen Tumor- oder Krebszelle umfasst, wobei die Tumor- oder Krebszelle eine primäre oder metastatische Tumorzelle ist, wobei die hybride Zelle auf ihrer Oberfläche B7 exprimiert;
b) In Kontakt bringen der hybriden Zelle mit einer menschlichen Immuneffektorzelle, wodurch eine menschliche geschulte Immuneffektorzelle erzeugt wird; und
c) In Kontakt bringen einer Zielzelle mit der menschlichen geschulten Immuneffektorzelle in der Gegenwart des Peptids, wobei die Lyse der Zielzelle das Peptid als ein antigenisches Peptid identifiziert.

2. Verfahren nach Anspruch 1, umfassend:
a) Bereitstellen einer Vielzahl von Zellen, wobei wenigstens 5 % der Zellen der Population fusionierte Zellen sind, die durch Fusion zwischen wenigstens einer menschlichen dendritischen Zelle und wenigstens einer menschlichen Tumor- oder Krebszelle erzeugt werden, die ein Zelloberflächen-Antigen exprimiert, wobei die Tumor- oder Krebszelle eine primäre oder metastatische Tumorzelle ist, wobei die fusionierten Zellen (i) ein MHC-Klasse-II-Molekül, (ii) B7 und (iii) das Zelloberflächen-Antigen in Mengen exprimieren, die wirksam sind, eine Immunantwort zu stimulieren;
b) In Kontakt bringen einer Population von menschlichen T-Lymphozyten mit der Vielzahl von Zellen, wobei das Inkontaktbringen die Differenzierung von Effektorzell-Vorläuferzellen in der Population von T-Lymphozyten zu Effektorzellen verursacht, die zytotoxische T-Lymphozyten umfassen; und
c) In Kontakt bringen von Zielzellen mit den Effektorzellen in der Gegenwart des Peptids;
wobei die Lyse der Vielzahl von Zielzellen oder eines Teils davon das Peptid als ein antigenisches Peptid identifiziert, das durch die zytotoxischen T-Lymphozyten erkannt wird.

3. Verfahren nach Anspruch 1, wobei das Peptid ein Fragment eines Proteins ist, das durch die Krebszelle exprimiert wird.

4. Verfahren, umfassend die Schritte des Testens eines oder mehrerer Peptide auf antigenische Aktivität nach Anspruch 1, Identifizieren wenigstens eines der Peptide als ein antigenisches Peptid und Verwenden des antigenischen Peptids oder eines Polypeptids, von dem das Peptid ein Fragment ist, bei der Herstellung eines Medikaments zur Verwendung beim Induzieren einer Immunantwort in einem Patienten.

5. Verfahren nach Anspruch 4, wobei das Peptid aus einem Polypeptid abgeleitet ist, das aus der Gruppe ausgewählt ist, die aus carzinoembryonalem Antigen, prostataspezifischem Antigen, HER2/neu, CA-125 und Mucin-1 (MUC1) besteht.

## Revendications

1. Procédé de test d'un peptide pour observer une activité antigénique, le procédé comprenant les étapes consistant à :
(a) se procurer une cellule hybride comprenant un produit de fusion d'une cellule dendritique humaine et d'une cellule tumorale ou cancéreuse humaine, ladite cellule tumorale ou cancéreuse étant une cellule tumorale primaire ou métastatique, ladite cellule hybride exprimant B7 à sa surface ;
(b) amener la cellule hybride en contact avec une cellule effectrice immune humaine, pour produire ainsi une cellule effectrice immune éduquée humaine ; et
(c) amener une cellule cible en contact avec ladite cellule effectrice immune éduquée humaine en présence du peptide, la lyse de ladite cellule cible identifiant le peptide en tant qu'un peptide antigénique.

2. Procédé selon la revendication 1, comprenant les étapes consistant à :
(a) se procurer une pluralité de cellules, au moins 5 % des cellules de ladite population étant des cellules fusionnées générées par fusion entre au moins une cellule dendritique humaine et au moins une cellule tumorale ou cancéreuse humaine qui exprime un antigène de surface cellulaire, ladite cellule tumorale ou cancéreuse étant une cellule tumorale primaire ou métastatique, lesdites cellules fusionnées exprimant, en des quantités efficaces pour stimuler une réponse immunitaire, (i) une molécule du CMH de classe II, (ii) B7, et (iii) l'antigène de surface cellulaire,
(b) amener une population de lymphocytes T humains en contact avec la pluralité de cellules, la mise en contact provoquant une différenciation de cellules précurseurs des cellules effectrices dans la population des lymphocytes T en des cellules effectrices comprenant les lymphocytes T cytotoxiques ; et
(c) amener une pluralité de cellules cibles en contact avec les cellules effectrices en présence du peptide,
la lyse de la pluralité de cellules cibles ou une partie de celles-ci identifiant le peptide en tant qu'un peptide antigénique qui est reconnu par les lymphocytes T cytotoxiques.

3. Procédé selon la revendication 1, dans lequel le peptide est un fragment d'une protéine exprimée par la cellule cancéreuse.

4. Procédé comprenant les étapes consistant à tester un ou plusieurs peptides pour observer une activité antigénique selon la revendication 1, identifier au moins l'un desdits peptides en tant que peptide antigénique, et utiliser ledit peptide antigénique ou un polypeptide dont ledit peptide antigénique est un fragment dans la fabrication d'un médicament pour une utilisation dans l'induction d'une réponse immunitaire chez un sujet.

5. Procédé selon la revendication 4, dans lequel ledit peptide est issu d'un polypeptide choisi dans le groupe constitué d'un antigène carcino-embryonnaire, d'un antigène spécifique de la prostate, de HER2/neu, de CA-125 et de mucine-1 (MUC1).
